(19) **European Patent Office** Europäisches Patentamt / European Patent Office / Office européen des brevets

(11) **EP 3 985 025 A1**

(12) **EUROPEAN PATENT APPLICATION**
published in accordance with Art. 153(4) EPC

(43) Date of publication:
**20.04.2022 Bulletin 2022/16**

(21) Application number: **20821874.3**

(22) Date of filing: **22.05.2020**

(51) International Patent Classification (IPC):
**C07K 16/28** (2006.01)    **A61P 35/00** (2006.01)
**A61K 39/00** (2006.01)

(52) Cooperative Patent Classification (CPC):
**A61K 39/00; A61P 35/00; C07K 16/28**

(86) International application number:
**PCT/KR2020/006705**

(87) International publication number:
**WO 2020/251187 (17.12.2020 Gazette 2020/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.06.2019 KR 20190069931**

(71) Applicants:
• **Green Cross Corporation**
**Yongin-si, Gyeonggi-do 16924 (KR)**
• **Mogam Institute for Biomedical Research**
**Yongin-si, Gyeonggi-do 16924 (KR)**

(72) Inventors:
• **PARK, Hye-Young**
Yongin-si
Gyeonggi-do 16924 (KR)
• **SONG, Eun Jung**
Yongin-si
Gyeonggi-do 16924 (KR)
• **LEE, Eun Hee**
Yongin-si
Gyeonggi-do 16924 (KR)
• **YUM, Hye In**
Yongin-si
Gyeonggi-do 16924 (KR)

• **NAM, Hye Mi**
Yongin-si
Gyeonggi-do 16924 (KR)
• **KIM, Mun Kyung**
Yongin-si
Gyeonggi-do 16924 (KR)
• **LEE, Jee Won**
Yongin-si
Gyeonggi-do 16924 (KR)
• **SHEEN, Joong Hyuk**
Yongin-si
Gyeonggi-do 16924 (KR)
• **HUR, Min Kyu**
Yongin-si
Gyeonggi-do 16924 (KR)
• **LIM, So Jung**
Yongin-si
Gyeonggi-do 16924 (KR)
• **LIM, Ok Jae**
Yongin-si
Gyeonggi-do 16924 (KR)
• **LIM, Yang Mi**
Yongin-si
Gyeonggi-do 16924 (KR)
• **WON, Jong Hwa**
Yongin-si
Gyeonggi-do 16924 (KR)

(74) Representative: **Mewburn Ellis LLP**
**Aurora Building**
**Counterslip**
**Bristol BS1 6BX (GB)**

(54) **ANTIBODY TO TIGIT AND USE THEREOF**

(57)    The present invention relates to: an antibody to T cell Immunoreceptor with Ig and Tyrosine-Based Inhibitory Motif Domains (TIGIT), or an antigen-binding fragment thereof; a nucleic acid encoding same; a vector carrying the nucleic acid; a cell transformed with the vector; a method for producing the antibody or the antigen-binding fragment thereof; and a composition and a composition for combined administration, which comprise same and are for preventing or treating cancer.

EP 3 985 025 A1

Fig. 1

**Description**

**Technical Field**

The present invention relates to an antibody that specifically binds to TIGIT (T-cell immunoreceptor with Ig and tyrosine-based inhibitory motif domains), an antigen-binding fragment thereof, a nucleic acid encoding the same, a vector comprising the nucleic acid, a cell transformed with the vector, a method of producing the antigen and antigen-binding fragment thereof, a composition for preventing or treating cancer comprising the same, and a composition for preventing or treating cancer comprising the same for administration in combination with another therapeutic agent.

**Background Art**

There are various cases involving cancer tissues, specifically, immune cells may be incapable of accessing cancer tissues, or immune cells may be capable of penetrating into the cancer tissues but the immune response may be suppressed by the cancer tissues. The human immune system has an immune checkpoint system to suppress a hyperimmune response caused by excessive proliferation of T-cells. An immune checkpoint blockade targeting an immune checkpoint protein involved in the immune checkpoint does not directly target cancer cells, but causes CTL (cytotoxic T cells) to eliminate cancer cells by restoring the activity of TIL (tumor-infiltrating lymphocytes), in particular, CTL, which are located around the cancer tissues, but the activity thereof is reduced by immunosuppressive factors (CD80, TIGIT) expressed by cancer cells. In this case, the immune checkpoint blockade blocks inhibitory signaling by preventing binding between T-cell inhibition receptors and inhibitory factors on the surface of cancer cells, and enables effective other stimulatory signaling, thus ultimately having an effect of increasing the activity of CTL.

Administration of a large number of conventional chemotherapeutic agents inevitably should be stopped due to the severe side effects thereof. However, immune checkpoint blockers exhibit clinical superiority in terms of overall survival, progression-free survival, and the like, and cause only mild side effects. In addition, conventional chemotherapeutic agents cannot be administered when the same cancer recurs because of acquired resistance to the therapeutic agent, whereas an anticancer immune response due to the administration of an immune checkpoint blocker causes a memory response upon recurrence of the same cancer, enabling rapid eradication of cancer cells.

Regarding the immune checkpoint blocker, ipilimumab, which is a monoclonal antibody specific for the immune checkpoint receptor CTLA-4 (cytotoxic T-lymphocyte associated antigen-4), has shown effectiveness in treating metastatic malignant melanoma. In addition, monoclonal antibodies specific for PD-1 (programmed cell death-1) and PD-L1 (programmed death ligand-1), which is a ligand for PD-1, are being developed. Representative examples thereof include nivolumab, pembrolizumab, avelumab, atezolizumab, durvalumab and the like. The effects of PD-1 or PD-L1 inhibitors are found in various tumors as well as in malignant melanoma.

Meanwhile, TIGIT (=Vstm-3, WUCAM) is a co-inhibitory molecule whose expression is induced when NK cells and T cells are activated, like PD-1. Cancer cells can escape attack from T cells by inhibiting T cells by overexpressing ligands for TIGIT. An anti-TIGIT antibody is predicted to prevent cancer progression by restoring the immune function of cancer patients by blocking the suppression of immune activity through TIGIT present in cancer cells.

TIGIT is induced and expressed in CD8+CTL and CD4+ T helper cells, similar to PD-1, and is continuously expressed along with PD-1 in FoxP3+ Treg, which plays a leading role in suppressing the immune response in cancer tissues, and is induced and expressed in higher levels when Treg is activated.

Several types of anti-TIGIT antibodies have been reported to date (US Patent Application Publication No. 2017-0088613, etc.), but research on specific mechanisms is still insufficient, and antibodies having efficacy to an extent enabling use as actual therapeutic agents have not been developed. So, demand for a TIGIT-specific antibody having high efficacy is still increasing.

Against this technical background, the present inventors endeavored to develop antibodies that specifically bind to TIGIT. As a result, the present inventors developed an anti-TIGIT antibody that binds to TIGIT with high affinity, and found that this anti-TIGIT antibody acts as an effective immuno-oncology agent. Based thereon, the present invention has been completed.

**Summary of the Invention**

It is one object of the present invention to provide a novel antibody to TIGIT or an antigen-binding fragment thereof.

It is another object of the present invention to provide a nucleic acid encoding the antibody or an antigen-binding fragment thereof.

It is another object of the present invention to provide a vector comprising the nucleic acid, a cell transformed with the vector, and a method for producing the same.

It is another object of the present invention to provide a composition for preventing or treating cancer comprising the antibody or an antigen-binding fragment thereof.

It is another object of the present invention to provide a composition for preventing or treating cancer by administering the antibody or an antigen-binding fragment thereof in combination with another drug.

To achieve the above objects, the present invention provides an antibody or an antigen-binding fragment thereof specifically binding to TIGIT (T-cell immunoreceptor with Ig and tyrosine-based inhibitory motif domains) comprising a heavy-chain CDR1 represented by SEQ ID NO: 3, 6, 9 or 12, a heavy-chain CDR2 represented by SEQ ID NO: 4, 7 or 10, a heavy-chain CDR3 represented by SEQ ID NO: 5, 8, 11 or 13, a light-chain CDR1 represented by SEQ ID NO: 14, 17, 20, 23 or 25, a light-chain CDR2 represented by SEQ ID NO: 15, 18, 21 or 26, and a light-chain CDR3 represented by SEQ ID NO: 16, 19, 22, 24 or 27.

The present invention also provides a nucleic acid encoding the antibody or an antigen-binding fragment thereof.

The present invention also provides a vector comprising the nucleic acid.

The present invention also provides a cell transformed with the vector.

The present invention also provides a method of producing the antigen or antigen-binding fragment thereof comprising (a) culturing the cell and (b) recovering an antibody or antigen-binding fragment thereof from the cultured cell.

The present invention also provides a composition for preventing or treating cancer comprising the antibody or antigen-binding fragment thereof as an active ingredient.

The present invention also provides a composition for preventing or treating cancer using the antibody or antigen-binding fragment thereof in combination with another anticancer therapy.

**Brief Description of Drawings**

FIG. 1 shows the result of a test on the binding capacity of a scFv-form anti-TIGIT antibody.

FIG. 2 shows the result of a test on the binding capacity of an IgG-type anti-TIGIT antibody.

FIG. 3 shows the results of a blocking assay for TIGIT and the ligand thereof, poliovirus receptor (PVR), performed on 11 anti-TIGIT antibodies.

FIG. 4 shows the affinity of the anti-TIGIT antibody, measured by Octet.

FIG. 5 shows the affinity of the anti-TIGIT antibody measured by BIACORE.

FIG. 6 shows the binding capacity between TIGIT expressed on the cell surface and TIGIT candidate antibodies.

FIG. 7 shows the competitiveness of the TIGIT candidate antibody to PVR, which is a ligand of TIGIT.

FIG. 8 shows the result of an assay for T-cell activation due to the binding inhibition effect between TIGIT and PVR by the TIGIT candidate antibody.

FIG. 9 shows the results of a cross-reaction to determine whether or not TIGIT candidate antibodies are capable of binding to TIGIT in mice, monkeys and humans.

FIG. 10 shows the result of confirmation that the function of the regulatory T cells is inhibited by the TIGIT candidate antibody, using co-culture of regulatory T cells and CFSE-labeled responder cells.

FIG. 11 shows the result of confirming the anticancer effect of the TIGIT candidate antibody.

FIGS. 12a and 12b show results of analysis of changes in T-cell proliferation and IFN-$\gamma$ secretion when treated with the specified antibodies in a combined lymphocyte reaction using dendritic cells and allogeneic T cells.

FIG. 13 shows the result of analyzing the proportion of cells expressing each activation marker upon treatment of the regulatory T cells of a healthy donor with the antibody specified.

FIG. 14 shows the result of analyzing the proportion of cells expressing exhaustion markers in CD8-positive T cells and regulatory T cells of a multiple myeloma patient.

FIG. 15 shows the result of analyzing the change in IFN-γ secretion upon treatment of PBMCs from a multiple myeloma patient with the specified antibody.

FIG. 16 shows the result of analyzing the expression of PVR in the HT29 cell line.

**Detailed Description and Preferred Embodiments of the Invention**

Unless defined otherwise, all technical and scientific terms used herein have the same meanings as those appreciated by those skilled in the field to which the present invention pertains. In general, the nomenclature used herein is well-known in the art and is ordinarily used.

In one aspect, the present invention is directed to an antibody or an antigen-binding fragment thereof specifically binding to TIGIT (T-cell immunoreceptor with Ig and tyrosine-based inhibitory motif domains), wherein the antibody or an antigen-binding fragment thereof comprising a heavy-chain CDR1 represented by SEQ ID NO: 3, 6, 9 or 12, a heavy-chain CDR2 represented by SEQ ID NO: 4, 7 or 10, a heavy-chain CDR3 represented by SEQ ID NO: 5, 8, 11 or 13, a light-chain CDR1 represented by SEQ ID NO: 14, 17, 20, 23 or 25, a light-chain CDR2 represented by SEQ ID NO: 15, 18, 21 or 26, and a light-chain CDR3 represented by SEQ ID NO: 16, 19, 22, 24 or 27.

The TIGIT comprises the sequence of SEQ ID NO: 1.

| Human TIGIT (SEQ ID NO: 1) |
| --- |
| MGWCLLLIWAQGLRQAPLASGMMTGTIETTGNISAEKGGSIILQCHLSSTTAQVTQVNWEQQDQLL AICNADLGWHISPSFKDRVAPGPGLGLTLQSLTVNDAGEYFCIYHTYPDGTYTGRIFLEVLESSVAEHG ARFQIPLLGAMAATLVVICTAVIVVVALTRKKKALRIHSVEGDLRRKSAGQEEWSPSAPSPPGSCVQAE AAPAGLCGEQRGEDCAELHDYFNVLSYRSLGNCSFFTETG |

As used herein, the term "antibody" refers to an anti-TIGIT antibody that specifically binds to TIGIT. The scope of the present invention comprises not only a complete antibody specifically binding to TIGIT but also an antigen-binding fragment of the antibody molecule.

The antibody of the present invention comprises, but is not limited to, monoclonal antibodies, multispecific antibodies, human antibodies, humanized antibodies, chimeric antibodies, single-chain Fvs (scFVs), single-chain antibodies, Fab fragments, F(ab') fragments, disulfide-bond Fvs (sdFVs), anti-idiotypic (anti-Id) antibodies, epitopebinding fragments of such antibodies, and the like.

The term "monoclonal antibody" refers to an antibody to a single determinant of an antigen and is an identical antibody, which is obtained from a population of substantially homogeneous antibodies, that is, an identical antibody constituting the population, excluding possible naturally occurring mutations that may be present in trivial amounts.

The term "epitope" refers to a protein determinant to which an antibody is capable of specifically binding. Epitopes usually consist of a group of chemically active surface molecules, such as amino acid or sugar side chains, and generally have not only specific three-dimensional structural characteristics but also specific charge characteristics. Three-dimensional epitopes are distinguished from non-three-dimensional epitopes in that a bond to the former is broken in the presence of a denatured solvent, while a bond to the latter is not broken.

The term "complete antibody" refers to a structure having two full-length light chains and two full-length heavy chains, wherein each light-chain is linked to a corresponding heavy-chain by a disulfide bond. The heavy-chain constant region has gamma (γ), mu (μ), alpha (α), delta (δ) and epsilon (ε) types, and is subclassified into gamma 1 (γ1), gamma 2 (γ2), gamma 3 (γ3), gamma 4 (γ4), alpha 1 (α1) and alpha 2 (α2). The light-chain constant region has kappa (κ) and lambda (λ) types.

The antigen-binding fragment of an antibody or antibody fragment is a fragment that has antigen-binding capability, and includes Fab, F(ab'), F(ab')2, Fv and the like. Among the antibody fragments, Fab refers to a structure including a variable region of each of the heavy chain and the light chain, the constant region of the light chain, and the first constant domain (CH1) of the heavy chain, each having one antigen-binding site. Fab' is different from Fab in that it further includes a hinge region including at least one cysteine residue at the C-terminus of the CH1 domain of the heavy chain. F(ab')2 is created by a disulfide bond between cysteine residues in the hinge region of Fab'. Fv is the minimal antibody fragment having only a heavy-chain variable region and a light-chain variable region. Two-chain Fv is a fragment in which the variable region of the heavy chain and the variable region of the light chain are linked by a non-covalent bond, and single-chain Fv (scFv) is a fragment wherein the variable region of the heavy chain and the variable region of the light chain are generally linked by a covalent bond via a peptide linker therebetween, or are directly linked at the C-terminus, forming a dimer-shaped structure, like the two-chain Fv. Such antibody fragments may be obtained using proteases (e.g. Fab can be obtained by restriction-cleaving the complete antibody with papain, and the F(ab')2 fragment can be obtained by cleaving the complete antibody with pepsin), and may be also prepared using genetic recombination techniques.

The "Fv" fragment is an antibody fragment comprising complete antibody recognition and binding sites. Such a region includes a dimer that consists of one heavy-chain variable domain and one light-chain variable domain substantially tightly covalently linked to each other, for example, through scFv.

A "Fab" fragment comprises a variable domain and a constant domain of the light-chain and a variable domain and a first constant domain (CH1) of the heavy chain. A F(ab')$_2$ antibody fragment generally includes a pair of Fab fragments covalently linked near the carboxyl terminal thereof via a hinge cysteine therebetween.

The "single-chain Fv" or "scFv" antibody fragment includes VH and VL domains of the antibody, wherein these domains are present in a single polypeptide chain. The Fv polypeptide may further include a polypeptide linker between the VH domain and the VL domain in order for the scFv to form a desired structure for antigen binding.

In one embodiment, the antibody of the present invention is in an Fv form (for example, scFv) or a complete antibody form. In addition, the heavy-chain constant region may be selected from gamma ($\gamma$), mu (u), alpha ($\alpha$), delta ($\delta$) and epsilon (c) isotypes. For example, the constant region may be gamma 1 (IgG1), gamma 3 (IgG3), or gamma 4 (IgG4) . The light-chain constant region may be kappa or lambda.

As used herein, the term "heavy chain" encompasses both a full-length heavy chain, which includes a variable domain (VH), comprising an amino acid sequence having a variable region sequence sufficient for imparting specificity to an antigen and three constant domains (CH1, CH2 and CH3), and a fragment thereof. As used herein, the term "light chain" encompasses both a full-length light chain, which includes a variable domain (VL) comprising an amino acid sequence having a variable region sequence sufficient for imparting specificity to an antigen and a constant domain (CL), and a fragment thereof.

A part of the heavy chain and/or light chain is identical to or homologous with the corresponding sequence in an antibody derived from a particular species or belonging to a particular antibody class or subclass, while the remainder of the chain(s) includes "chimeric" antibodies (immunoglobulins) which are identical to or homologous with corresponding sequences in an antibody derived from another species or belonging to another antibody class or subclass, as well as fragments of such antibody exhibiting the desired biological activity.

As used herein, the term "antibody variable domain" refers to the light- and heavy-chain regions of an antibody molecule comprising the amino acid sequences of a complementarity-determining region (CDR; i.e., CDR1, CDR2, and CDR3) and a framework region (FR). VH refers to a variable domain of the heavy chain. VL refers to a variable domain of the light chain.

The term "complementarity-determining region" (CDR; that is, CDR1, CDR2, and CDR3), refers to an amino acid residue of the antibody variable domain, which is necessary for antigen binding. Each variable domain typically has three CDR regions, identified as CDR1, CDR2, and CDR3. In one embodiment, the complementarity-determining region comprises a heavy-chain CDR1 represented by SEQ ID NO: 3, 6, 9 or 12, a heavy-chain CDR2 represented by SEQ ID NO: 4, 7 or 10, a heavy-chain CDR3 represented by SEQ ID NO: 5, 8, 11 or 13, a light-chain CDR1 represented by SEQ ID NO: 14, 17, 20, 23 or 25, a light-chain CDR2 represented by SEQ ID NO: 15, 18, 21 or 26, and a light-chain CDR3 represented by SEQ ID NO: 16, 19, 22, 24 or 27.

In the present invention, the antibody binding to TIGIT or antigen-binding fragment thereof may comprise a heavy-chain CDR1 of SEQ ID NO: 3, a heavy-chain CDR2 of SEQ ID NO: 4, a heavy-chain CDR3 of SEQ ID NO: 5, a light-chain CDR1 of SEQ ID NO: 14, a light-chain CDR2 of SEQ ID NO: 15, and a light-chain CDR3 of SEQ ID NO: 16;

a heavy-chain CDR1 of SEQ ID NO: 6, a heavy-chain CDR2 of SEQ ID NO: 7, a heavy-chain CDR3 of SEQ ID NO: 8, a light-chain CDR1 of SEQ ID NO: 17, a light-chain CDR2 of SEQ ID NO: 18, and a light-chain CDR3 of SEQ ID NO: 19;

a heavy-chain CDR1 of SEQ ID NO: 9, a heavy-chain CDR2 of SEQ ID NO: 10, a heavy-chain CDR3 of SEQ ID NO: 11, a light-chain CDR1 of SEQ ID NO: 20, a light-chain CDR2 of SEQ ID NO:21, and a light-chain CDR3 of SEQ ID NO: 22;

a heavy-chain CDR1 of SEQ ID NO: 9, a heavy-chain CDR2 of SEQ ID NO: 10, a heavy-chain CDR3 of SEQ ID NO: 11, a light-chain CDR1 of SEQ ID NO: 23, a light-chain CDR2 of SEQ ID NO: 21, and a light-chain CDR3 of SEQ ID NO: 24; or

a heavy-chain CDR1 of SEQ ID NO: 12, a heavy-chain CDR2 of SEQ ID NO: 10, a heavy-chain CDR3 of SEQ ID NO: 13, a light-chain CDR1 of SEQ ID NO: 25, a light-chain CDR2 of SEQ ID NO: 26, and a light-chain CDR3 of SEQ ID NO: 27.

The term "framework region" (FR) refers to a variable domain residue other than a CDR residue. Each variable domain typically has four FRs, identified as FR1, FR2, FR3, and FR4.

The TIGIT antibody may have a monovalent or divalent form, and may comprise single or double chains. Functionally, the binding affinity of the TIGIT antibody ranges from $10^{-5}$ M to $10^{-12}$ M. For example, the binding affinity of the TIGIT antibody may range from $10^{-6}$ M to $10^{-12}$ M, $10^{-7}$ M to $10^{-12}$ M, $10^{-8}$ M to $10^{-12}$ M, $10^{-9}$ M to $10^{-12}$ M, $10^{-5}$ M to $10^{-11}$ M, $10^{-6}$ M to $10^{-11}$ M, $10^{-7}$ M to $10^{-11}$ M, $10^{-8}$ M to $10^{-11}$ M, $10^{-9}$ M to $10^{-11}$ M, $10^{-10}$ M to $10^{-11}$ M, $10^{-5}$ M to $10^{-10}$ M, $10^{-6}$ M to $10^{-10}$ M, $10^{-7}$ M to $10^{-10}$ M, $10^{-8}$ M to $10^{-10}$ M, $10^{-9}$ M to $10^{-10}$ M, $10^{-5}$ M to $10^{-9}$ M, $10^{-6}$ M to $10^{-9}$ M, $10^{-7}$ M to $10^{-9}$ M, $10^{-8}$ M to $10^{-9}$ M, $10^{-5}$ M to $10^{-8}$ M, $10^{-6}$ M to $10^{-8}$ M, $10^{-7}$ M to $10^{-8}$ M, $10^{-5}$ M to $10^{-7}$ M, $10^{-6}$ M to $10^{-7}$ M, or $10^{-5}$ M to $10^{-6}$ M.

The antibody binding to TIGIT or an antigen-binding fragment thereof may comprise at least one heavy-chain variable region selected from the group consisting of SEQ ID NOS: 28 to 32. In addition, the antibody binding to TIGIT or an antigen-binding fragment thereof may comprise at least one light-chain variable region selected from the group consisting of SEQ ID NOS: 34 to 38.

Specifically, the antibody binding to TIGIT or an antigen-binding fragment thereof comprises: the heavy-chain variable region of SEQ ID NO: 28 and the light-chain variable region of SEQ ID NO: 34; the heavy-chain variable region of SEQ ID NO: 29 and the light-chain variable region of SEQ ID NO: 35; the heavy-chain variable region of SEQ ID NO: 30 and the light-chain variable region of SEQ ID NO: 36; the heavy-chain variable region of SEQ ID NO: 31 and the light-chain variable region of SEQ ID NO: 37; or the heavy-chain variable region of SEQ ID NO: 32 and the light-chain variable region of SEQ ID NO: 38.

"Phage display" is a technique for displaying a mutant polypeptide as a fusion protein with at least a part of a coat protein on the surface of the particle of a phage, for example a fibrous phage. The usefulness of phage display is to rapidly and efficiently classify sequences that bind to target antigens with high affinity in large libraries of randomized protein mutants. Displaying peptides and protein libraries on phages has been used to screen millions of polypeptides in order to identify polypeptides with specific binding properties.

Phage display technology has offered a powerful tool for producing and screening novel proteins that bind to specific ligands (e.g., antigens). Using phage display technology, large libraries of protein mutants can be generated, and sequences binding with high affinity to target antigens can be rapidly classified. The nucleic acid encoding mutant polypeptides is fused with a nucleic acid sequence encoding a viral coat protein, e.g. a gene III or gene VIII protein. A monophasic phage display system, in which a nucleic acid sequence encoding a protein or polypeptide is fused with a nucleic acid sequence encoding a part of a gene III protein, has been developed. In the monophasic display system, a fused gene is expressed at a low level, and a wildtype gene III protein is also expressed, and thus particle infectivity is maintained.

It is important to demonstrate the expression of peptides on the fibrous phage surface and the expression of functional antibody fragments in the peripheral cytoplasm of *E. coli* for the development of antibody phage display libraries. Libraries of antibody- or antigen-binding polypeptides are produced through a number of methods, for example, methods of modifying a single gene by inserting a random DNA sequence or cloning a related gene sequence. Libraries can be screened regarding the expression of antibody- or antigen-binding proteins having desired characteristics.

Phage display technology has several advantages over conventional hybridomas and recombinant methods for producing antibodies having desired characteristics. This technique provides the generation of large antibody libraries with a variety of sequences within a short time without using animals. The production of hybridomas and the production of humanized antibodies may require a production time of several months. In addition, since no immunity is required, the phage antibody libraries can generate antibodies against antigens that are toxic or have low antigenicity. The phage antibody libraries can also be used to produce and identify novel therapeutic antibodies.

Techniques for generating human antibodies from immunized or non-immunized human germline sequences or naive B cell Ig repertoires using phage display libraries can be used. Naive or non-immunogenic antigen-binding libraries can be produced using various lymphatic tissues.

Techniques for identifying and separating high-affinity antibodies from phage display libraries are important for the separation of new therapeutic antibodies. The separation of high-affinity antibodies from libraries depends on the size of the libraries, the production efficiency in bacterial cells, and the variety of libraries. The size of the libraries is reduced by improper folding of the antibody- or antigen-binding protein and inefficient production due to the presence of the stop codon. Expression in bacterial cells may be inhibited by improper folding of the antibody- or antigen-binding domain. The expression can be improved by alternately mutating residues on the surface of the variable/constant interfaces or the selected CDR residues. The sequence of the framework region is an element that enables proper folding when generating antibody phage libraries in bacterial cells.

It is important to generate various libraries of antibody- or antigen-binding proteins in the separation of high-affinity antibodies. CDR3 regions have often been found to participate in antigen binding. Since the CDR3 region in the heavy chain varies considerably with regard to size, sequence and structural/dimensional morphology, various libraries can be produced using the same.

Also, diversity can be created by randomizing the CDR regions of variable heavy and light chains using all 20 amino acids at each position. The use of all 20 amino acids results in the production of antibody sequences having increased diversity and an increased chance of identifying new antibodies.

The antibody or antibody fragment of the present invention may comprise the sequence of the anti-TIGIT antibody mentioned herein as well as biological equivalents thereto, as long as it can specifically recognize TIGIT. For example, additional variations can be made to the amino acid sequence of the antibody in order to further improve the binding affinity and/or other biological properties of the antibody. Such variations include, for example, deletion, insertion and/or substitution of the amino acid sequence residues of the antibody. Such amino acid variations are based on the relative similarity of amino acid side chain substituents, such as the hydrophobicity, hydrophilicity, charge, and size thereof. It can be seen through analysis of the size, shape and type of amino acid side chain substituents that all of arginine, lysine, and histidine are positively charged residues; alanine, glycine, and serine have similar sizes; and phenylalanine, tryptophan, and tyrosine have similar structure. Thus, based on these considerations, arginine, lysine, and histidine; alanine, glycine, and serine; and phenylalanine, tryptophan, and tyrosine are considered to be biologically functional equivalents.

Taking into consideration variations having biologically equivalent activity, the antibody or a nucleotide molecule encoding the same according to the present invention is interpreted to comprise a sequence having substantial identity with the sequence set forth in the sequence number. The term "substantial identity" means that a sequence has a homology of at least 90%, most preferably a homology of at least 95%, at least 96%, at least 97%, at least 98%, or at least 99%, when aligning the sequence of the present invention with any other sequence so as to correspond to each other as closely as possible and analyzing the aligned sequence using algorithms commonly used in the art. Alignment methods for sequence comparison are well-known in the art. The NCBI Basic Local Alignment Search Tool (BLAST) is accessible through NCBI or the like, and can be used in conjunction with sequence analysis programs such as blastp, blastm, blastx, tblastn and tblastx over the Internet. BLAST is available at www.ncbi.nlm.nih.gov/BLAST/. A method of comparing sequence homology using this program can be found at www.ncbi.nlm.nih.gov/BLAST/blast_help.html.

Based on this, the antibody or antigen-binding fragment thereof according to the present invention can have homology of 90%, 91%, 92%, 93%, 94%, 95%, 96%, 97%, 98%, 99%, or more with the sequence disclosed herein or the entirety thereof. Homology can be determined through sequence comparison and/or alignment by methods known in the art. For example, the percentage sequence homology of the nucleic acid or protein according to the present invention can be determined using a sequence comparison algorithm (i.e., BLAST or BLAST 2.0), manual alignment, or visual inspection.

In another aspect, the present invention is directed to a nucleic acid encoding the antibody or an antigen-binding fragment thereof.

By isolating the nucleic acid encoding the antibody or antigen-binding fragment thereof according to the present invention, an antibody or antigen-binding fragment thereof can be produced in a recombinant manner. The nucleic acid is isolated and inserted into a replicable vector, followed by further cloning (amplification of DNA) or further expression. Based thereon, in another aspect, the present invention is directed to a vector comprising the nucleic acid.

The term "nucleic acid" is intended to encompass both DNA (gDNA and cDNA) and RNA molecules, and a nucleotide, which is the basic constituent unit of nucleic acids, includes naturally derived nucleotides as well as analogues thereof, in which sugar or base moieties are modified. The sequence of the nucleic acid encoding heavy- and light-chain variable regions of the present invention can vary. Such variation includes addition, deletion, or non-conservative or conservative substitution of nucleotides.

The DNA encoding the antibody can be easily separated or synthesized using conventional procedures (for example, using an oligonucleotide probe capable of specifically binding to DNA encoding heavy and light chains of the antibody). A variety of vectors are obtainable. Vector components generally include, but are not limited to, one or more of the following components: signal sequences, replication origins, one or more marker genes, enhancer elements, promoters, and transcription termination sequences.

As used herein, the term "vector" refers to a means for expressing target genes in host cells, and includes plasmid vectors, cosmid vectors, and viral vectors such as bacteriophage vectors, adenovirus vectors, retroviral vectors, and adeno-associated viral vectors. The nucleic acid encoding the antibody in the vector is operably linked to a promoter.

The term "operably linked" means functional linkage between a nucleic acid expression regulation sequence (e.g., an array of the binding site of the promoter, signal sequence, or transcription regulator) and another nucleic acid sequence, and enables the regulation sequence to regulate transcription and/or translation of the other nucleic acid sequence.

When a prokaryotic cell is used as a host, it generally includes a potent promoter capable of conducting transcription (such as a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it includes a promoter derived from the genome of a mammalian cell (e.g., a metallothionein promoter, a β-actin promoter, a human hemoglobin promoter or a human muscle creatine promoter), or a promoter derived from a mammalian virus (e.g., an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter), and generally has a polyadenylation sequence as a transcription termination sequence.

Optionally, the vector may be fused with another sequence in order to facilitate purification of the antibody expressed thereby. The sequence to be fused therewith may include, for example, glutathione S-transferase (Pharmacia, USA), maltose-binding protein (NEB, USA), FLAG (IBI, USA), 6x His (hexahistidine; Qiagen, USA) and the like.

The vector comprises antibiotic resistance genes commonly used in the art as selectable markers, and examples thereof include genes conferring resistance to ampicillin, gentamycin, carbenicillin, chloramphenicol, streptomycin, kanamycin, geneticin, neomycin, and tetracycline.

In another aspect, the present invention is directed to a cell transformed with the above-mentioned vector. The cell used to produce the antibody of the present invention may be a prokaryote, yeast, or higher eukaryotic cell, but is not limited thereto.

Prokaryotic host cells such as *Escherichia coli,* strains of the genus *Bacillus,* such as *Bacillus subtilis* and *Bacillus thuringiensis, Streptomyces* spp., *Pseudomonas* spp. (for example, *Pseudomonas putida), Proteus mirabilis* and *Staphylococcus* spp. (for example, *Staphylococcus carnosus)* may be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing the antibody or antigen-binding fragment thereof comprising (a) culturing the cell; and (b) recovering an antibody or antigen-binding fragment thereof from the cultured cell.

The cells can be cultured in various media. Any commercially available medium can be used as a culture medium without limitation. All other essential supplements well-known to those skilled in the art may be included in appropriate concentrations. Culture conditions such as temperature and pH are those that are conventionally used with host cells selected for expression, as will be apparent to those skilled in the art.

The recovery of the antibody or antigen-binding fragment thereof can be carried out, for example, by centrifugation or ultrafiltration to remove impurities from and purify the resulting product using, for example, affinity chromatography. Other additional purification techniques, such as anion or cation exchange chromatography, hydrophobic interaction chromatography, and hydroxyapatite (HA) chromatography, may be used.

In another aspect, the present invention is directed to a composition for preventing or treating cancer or tumors comprising the antibody as an active ingredient.

For example, the present invention is directed to a pharmaceutical composition for preventing or treating cancer or tumors comprising (a) a pharmaceutically effective amount of the antibody to TIGIT or antigen-binding fragment thereof according to the present invention; and (b) a pharmaceutically acceptable carrier. In another aspect, the present invention is directed to a method for preventing or treating cancer or tumors comprising administering the antibody to TIGIT or an antigen-binding fragment thereof according to the present invention to a patient in an effective amount determined according to the requirements of the patent.

In the present invention, the terms "cancer" or "tumor" typically refer to or mean the physiological condition of a mammal characterized by uncontrolled cell growth/proliferation.

The composition uses the anti-TIGIT antibody or antigen-binding fragment thereof according to the present invention described above as an active ingredient, and thus redundant descriptions will be omitted.

As demonstrated in Examples given below, the antibody or antigen-binding fragment thereof according to the present invention binds to TIGIT with high affinity, and can be useful for treating cancer that evades anti-tumor T-cell activity.

The cancer or carcinoma that can be treated with the composition of the present invention is not particularly limited, and includes both solid cancer and blood cancer. Examples of such cancer include, but are not limited to, lymphoma, leukemia, and multiple myeloma.

The specific example according to the present invention demonstrated that TIGIT is expressed to a high level in regulatory T cells of multiple myeloma patients, and treatment with the antibody or antigen-binding fragment thereof according to the present invention resulted in a significant increase in IFN-γ secretion upon anti-CD3/anti-CD28 stimulation to PBMC of multiple myeloma patients.

For example, the cancer may be selected from the group consisting of skin cancer such as melanoma, liver cancer, hepatocellular carcinoma, gastric cancer, breast cancer, lung cancer, ovarian cancer, bronchial cancer, nasopharyngeal cancer, laryngeal cancer, pancreatic cancer, bladder cancer, colorectal cancer, colon cancer, uterine cervical cancer, brain cancer, prostate cancer, bone cancer, thyroid cancer, parathyroid cancer, renal cancer, esophageal cancer, biliary tract cancer, testicular cancer, rectal cancer, head and neck cancer, cervical cancer, ureteral cancer, osteosarcoma, neurocytoma, fibrosarcoma, rhabdomyosarcoma, astrocytoma, neuroblastoma, and neuroglioma, but is not limited thereto.

The specific example according to the present invention demonstrated that when a human colorectal cancer cell xenograft model was treated with the antibody or antigen-binding fragment thereof according to the present invention, an excellent effect of inhibiting tumor growth was obtained.

In another aspect, the present invention is directed to a composition for preventing or treating cancer by using the antibody or antigen-binding fragment thereof in combination with another anticancer therapy or administering the same in combination with another drug, for example, an anti-cancer agent.

For example, the present invention is directed to a composition for co-administration for preventing or treating cancer or tumors comprising (a) a pharmaceutically effective amount of the antibody to TIGIT or antigen-binding fragment thereof according to the present invention; and (b) a pharmaceutically acceptable carrier. In another aspect, the present invention is directed to a co-administration method for preventing or treating cancer or tumors comprising administering the antibody to TIGIT or antigen-binding fragment thereof according to the present invention to a patient in an effective amount as required by the patent.

The composition uses the anti-TIGIT antibody or antigen-binding fragment thereof according to the present invention described above as an active ingredient, and thus redundant descriptions will be omitted.

By administering other anticancer agents in addition to the antibody in combination therewith, it is possible to effectively target tumor cells overexpressing TIGIT and to increase anti-tumor T-cell activity and to thereby enhance the immune response targeting tumor cells.

The antibody may be used in combination with: other anti-neoplastic or immunogenic agents [e.g., attenuated cancer cells, tumor antigens (including recombinant proteins, peptides, and carbohydrate molecules), antigen transfer cells, for example, tumor-derived antigens or nucleic-acid-pulsed dendritic cells, immunostimulating cytokines (e.g., IL-2, IFNα2, and GM-CSF), and cells transfected with genes encoding immunostimulating cytokines (including for example but not limited to GM-CSF)]; standard cancer therapy (e.g. chemotherapy, radiotherapy or surgery); or other antibodies (including but not limited to antibodies other than TIGIT antibodies, VEGF, EGFR, Her2/neu, VEGF receptors, other growth factor receptors, CD20, CD40, CTLA-4, OX-40, 4-IBB, and ICOS).

The drug, for example, an anticancer agent, may be an immune checkpoint inhibitor, but is not limited thereto.

In another aspect, the present invention is directed to a composition for preventing or treating cancer using the antibody or antigen-binding fragment thereof in combination with an immune checkpoint inhibitor.

In the present invention, the immune checkpoint inhibitor refers to an agent that is capable of inducing T-cell activation by blocking movement of the T-cell inhibitory signal to the site where antigen-presenting cells (APCs) meet with immune cells, for example, T cells. The immune checkpoint inhibitor may be, for example, a drug targeting PD-1, PD-L1, or CTLA-4, but is not limited thereto.

The immune checkpoint inhibitor may specifically be an anti-CTLA-4 antibody, an anti-PD-1 antibody, or an anti-PD-L1 antibody, but is not limited thereto. Specifically, the immune checkpoint inhibitor may be ipilimumab, nivolumab, pembrolizumab, atezolizumab, avelumab, durvalumab or the like, but is not limited thereto.

A specific example of the present invention showed that when the anti-TIGIT antibody or antigen-binding fragment thereof is used in combination with the anti-PD-1 antibody, pembrolizumab, the T cell reactivity can be increased, T-cell proliferation can be increased, and IFN-γ secretion can be increased, compared to when the anti-TIGIT antibody is used alone.

The term "used in combination", "combined use" or "co-administration" means that the anti-TIGIT antibody or antigen-binding fragment thereof and another drug, for example, an anticancer agent, can be administered simultaneously, sequentially, or in reverse order. The composition of the present invention may be administered as a single therapeutic agent or in combination with other therapeutic agents, and may be administered sequentially or simultaneously with a conventional therapeutic agent.

The cancer, which is the disease to which the composition can be applied, includes typical cancers that respond to immunotherapy, as well as cancers that have not been related with immunotherapy to date. Non-limiting examples of cancer that is the target of treatment include melanoma (e.g., metastatic malignant melanoma), kidney cancer (e.g., clear cell carcinoma), prostate cancer (e.g., hormone refractory prostate adenocarcinoma), pancreatic adenocarcinoma, breast cancer, colon cancer, lung cancer (e.g. non-small cell lung cancer), esophageal cancer, head and neck squamous cell carcinoma, liver cancer, ovarian cancer, cervical cancer, thyroid cancer, glioblastoma, glioma, leukemia, lymphoma, blood cancers such as multiple myeloma, and other neoplastic carcinomas. In addition, the cancer according to the present invention includes refractory or recurrent cancer, the growth of which can be inhibited using the antibody of the present invention.

Antibodies or antibody fragments can be used alone or in combination with vaccines to stimulate an immune response against pathogens, toxins, and self-antigens. Antibodies or antigen-binding fragments thereof can be used to stimulate an immune response against viruses that infect humans, and examples of such viruses include, but are not limited to, human immunodeficiency virus, hepatitis virus classes A, B and C, Epstein-Barr virus, human cytomegalovirus, human papilloma virus, and herpes viruses. Antibodies or antigen-binding fragments thereof can be used to stimulate an immune response to infection with bacterial or fungal parasites and other pathogens.

The pharmaceutically acceptable carrier comprised in the composition according to the present invention may be commonly used in preparations, and may include, but is not limited to, lactose, dextrose, sucrose, sorbitol, mannitol, starch, gum acacia, calcium phosphate, alginate, gelatin, calcium silicate, microcrystalline cellulose, polyvinylpyrrolidone, water, syrup, methylcellulose, methylhydroxybenzoate, propylhydroxybenzoate, talc, magnesium stearate, mineral oil, and the like. The composition according to the present invention may further comprise a lubricant, a wetting agent, a sweetener, a flavoring agent, an emulsifier, a suspension agent, a preservative, or the like, in addition to the ingredients described above.

The pharmaceutical composition according to the present invention may be administered orally or parenterally. The parenteral administration may be intravenous injection, subcutaneous injection, intramuscular injection, intraperitoneal injection, endothelial administration, topical administration, intranasal administration, pulmonary administration, rectal administration, or the like.

Upon oral administration, since proteins or peptides are digested, an oral composition should be coated with an active drug or formulated so as to protect the same from degradation in the stomach. In addition, the pharmaceutical composition may be administered using any device capable of delivering the active substance to target cells.

The suitable dose of the pharmaceutical composition according to the present invention may vary depending on factors such as the formulation method, administration method, and age, body weight, gender, pathological conditions, diet, administration time, administration route, excretion rate, and responsiveness of the patient, and a general physician of ordinary skill can easily determine and prescribe a dose effective for the desired treatment or prevention. For example, the daily dose of the pharmaceutical composition according to the present invention may be within the range of 0.0001 to 100 mg/kg. The term "pharmaceutically effective amount" as used herein may mean an amount sufficient to prevent or treat cancer.

The pharmaceutical composition according to the present invention may be prepared into a unit dose form, or may be incorporated into a multi-dose container through formulation using a pharmaceutically acceptable carrier and/or excipient according to a method that can be easily implemented by those skilled in the art to which the present invention pertains. Here, the formulation may be in the form of a solution, a suspension or an emulsion in an oil or aqueous medium, or may be in the form of an extract, a powder, a suppository, a granule, a tablet, or a capsule. The composition may further comprise a dispersant or a stabilizer.

**Example**

Hereinafter, the present invention will be described in more detail with reference to examples. However, it will be obvious to those skilled in the art that these examples are provided only for illustration of the present invention, and should not be construed as limiting the scope of the present invention.

**Example 1. Screening of TIGIT antibodies**

Human synthetic library phage was reacted in a tube coated with human TIGIT-His antigen for 2 hours. After the reaction, the reaction product was washed 4 times with washing buffer (PBS + 0.05% Tween 20) and reacted with elution buffer (1% BSA/0.1M glycine, pH 2.0) at room temperature for 10 minutes, and then the phage was recovered. XLI-Blue competent cells were infected with the recovered phage and incubated at 37°C for 1 hour. Then, the result was treated with 1 mL of a VCS M13 helper phage, incubated at 37°C for 1 hour, further treated with 80 mL of SB medium, 100 $\mu$l of kanamycin, and 100 $\mu$l of carbenicillin, and then incubated at 37°C for 20 hours. After incubation, the supernatant was recovered, the phage was precipitated with a PEG solution (20% PEG, 15% NaCl), re-suspension was performed with 2 ml of 1% BSA/PBS, and the result was used for the next panning.

The human antibody library was displayed on the phage surface to screen human TIGIT-specific human antibodies and then the concentration of the antigen, TIGIT, was changed, as the number of rounds increased, and panning was performed for a total of 3 rounds. As the number of times panning was performed increased, the concentration of TIGIT was decreased from 100 nM to 1 nM. The output titer of the phage is shown in Table 1.

[Table 1]

| Phage output titer | | | |
|---|---|---|---|
| Round | Antigen | Concentration (nM) | Output titer |
| 1 | TIGIT-HIS | 100nM | $5.0E+10^7$ |
| 2 | TIGIT-HIS | 50nM | $3.2E+10^6$ |

(continued)

| Phage output titer | | | |
|---|---|---|---|
| Round | Antigen | Concentration (nM) | Output titer |
| 3 | TIGIT-HIS | 1nM | 7.8E+10$^5$ |

Example 2. Determination of binding capacity to **TIGIT**

2.1 Determination of binding capacity of ScFv-type anti-TIGIT antibody

50 μl of a human TIGIT-FC antigen was added at a concentration of 3 μg/ml to an ELISA plate, incubated at 4°C overnight, and then blocked with 1% BSA in PBS at room temperature for 2 hours. A total of 16 scFv anti-TIGIT antibodies were serially diluted by 1/3 in PBS at a starting concentration of 80 nM, fed in an amount of 50 μl into a human TIGIT-Fc plate, and then allowed to react at room temperature for 2 hours. The result was washed 3 times with PBST (0.05% Tween 20 in PBS) and was reacted with 50 μl of anti-His-HRP diluted 1/1000 in PBS at room temperature for 1 hour. The result was washed 3 times with PBST (0.05% Tween 20 in PBS), and 50 μl of TMB solution was added thereto. The result was reacted at room temperature for 5 minutes, 50 μl of a TMB stop solution was added thereto, and the absorbance was measured at 450 nm with an ELISA leader.

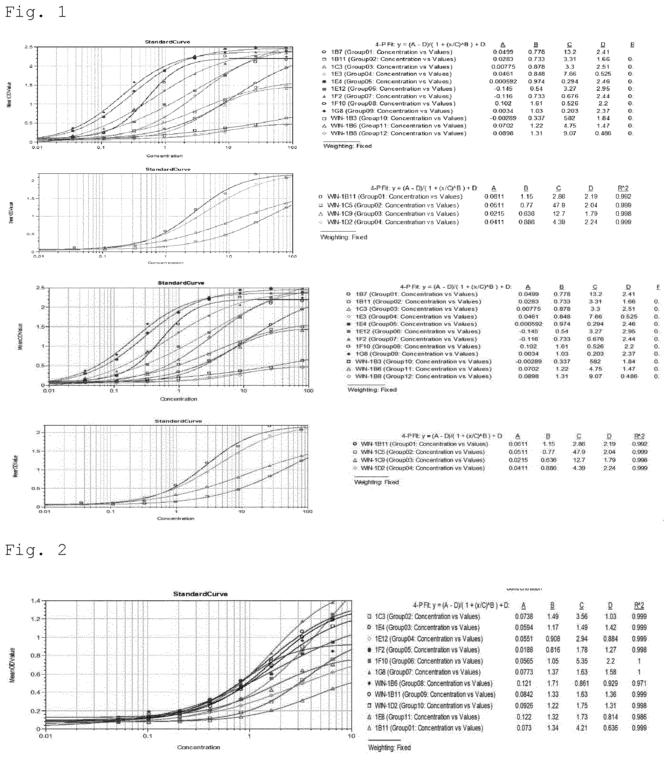

The results are shown in FIG. 1. A total of 11 clones (1C3, 1E4, 1E12, 1F2, 1F10, 1G8, WIN_1B6, WIN_1B11, WIN_1D2, 1E8, and 1B11) were selected based on the EC50 value.

2.2 Determination of binding affinity of IgG-type anti-TIGIT antibody

Among the scFv anti-human TIGIT antibodies, 11 types of antibodies having high binding affinity to human TIGIT were converted to IgG antibodies. 50 μl of the human TIGIT-Fc antigen was added at a concentration of 3 μg/ml to an ELISA plate, incubated at 4°C overnight, and then blocked with 1% BSA in PBS at room temperature for 2 hours. The total of 11 IgG-type anti-human TIGIT antibodies were serially diluted by 1/2 in PBS at a starting concentration of 6.6 nM, and 50 μl thereof was added to a human TIGIT-Fc plate and then allowed to react at room temperature for 2 hours. The result was washed 3 times with PBST (0.05% Tween 20 in PBS), and 50 μl of anti-His-HRP was diluted 1/1000 in PBS and then reacted at room temperature for 1 hour. The result was washed 3 times with PBST (0.05% Tween 20 in PBS), and 50 μl of a TMB solution was added thereto. The result was allowed to react at room temperature for 5 minutes, 50 μl of a TMB stop solution was added thereto, and the absorbance was measured at 450 nm in an ELISA leader. The results are shown in Table 2 and FIG. 2.

[Table 2]

| Clone | 1B11 | 1C3 | 1E4 | 1E12 | 1F2 | 1F10 | 1G8 | WIN-1B6 | WIN-1B1 1 | WIN-1D2 | 1E8 |
|---|---|---|---|---|---|---|---|---|---|---|---|
| EC50 (nM) | 4.21 | 3.56 | 1.49 | 2.94 | 1.78 | 5.35 | 1.63 | 0.86 | 1.63 | 1.75 | 1.73 |

It was found that 1E4, 1F2, 1G8, WIN-1D2, and 1E8 had a binding affinity of about 1 nM EC50.

Example 3. **Determination** of **competitiveness** of TIGIT **candidate antibody for PVR, ligand of** TIGIT

3.1 Human PVR expression and purification

The amino acid (SEQ ID NO: 2) of the ECD (extracellular domain) Gly27-Asn343 region of the human PVR sequence provided by GenBank AAH15542.1 was synthesized.

| Human PVR (Gly27-Asn343) SEQ ID NO: 2 |
|---|
| GDVVVQAPTQVPGFLGDSVTLPCYLQVPNMEVTHVSQLTWARHGESGSMAVFHQTQGPSYSESKRLEFVAA |
| RLGAELRNASLRMFGLRVEDEGNYTCLFVTFPQGSRSVDIWLRVLAKPQNTAEVQKVQLTGEPVPMARCVSTG |
| GRPPAQITWHSDLGGMPNTSQVPGFLSGTVTVTSLWILVPSSQVDGKNVTCKVEHESFEKPQLLTVNLTVYYPP |
| EVSISGYDNNWYLGQNEATLTCDARSNPEPTGYNWSTTMGPLPPFAVAQGAQLLIRPVDKPINTTLICNVTNA |
| LGARQAELTVQVKEGPPSEHSGMSRN |

PCR was performed with hPVR-F (5'-GGCCCAGGCGGCCGGCGA-3')/hPVR-R (5'-GGCCAGGCTGGCCGTTCC-3') primers, using the synthesized human PVR as a template, and then inserted into the pclw-huIgG4 vector using a Sfi I enzyme. 30 $\mu$g of light-chain DNA and heavy-chain DNA at a ratio of 1:1 was transiently expressed in 30 m$\ell$ of $2.5 \times 10^6$ Expi293F™ cells/ml using an ExpiFectamine 293 reagent and then incubated for 10 days. The cell culture solution (supernatant) was filtered using a 0.22 $\mu$m bottle top filter, and was then reacted with 200 $\mu$l of MabSelect Xtra beads while mixing in a bio-rotator for 2 hours. After incubation, the bead and antibody mixture was added to the protein A column. The protein A column was washed twice with 2 ml of binding buffer. A total of five elution fractions were collected using 200 $\mu$l of protein A elution buffer in the protein A column. The eluted antibodies were desalted by centrifugation at 1,000 rpm for 5 minutes using a Zeba spin desalting column.

3.2 Determination of competitiveness

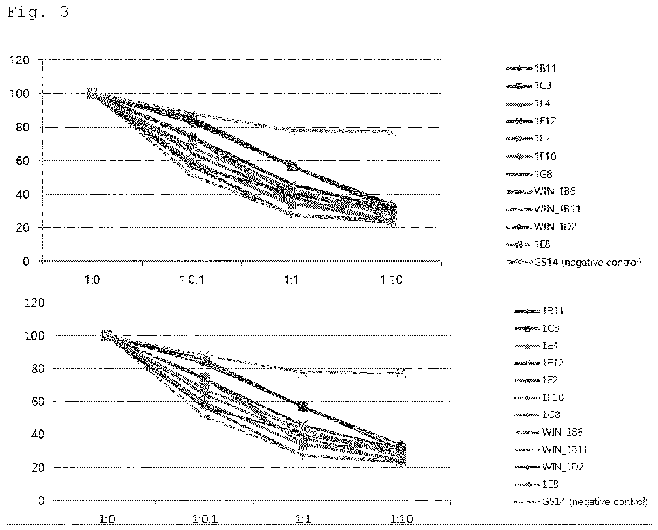

50 $\mu$l of a human TIGIT-Fc antigen was added at a concentration of 3 $\mu$g/ml to an ELISA plate, incubated at 4°C overnight, and blocked with 1% BSA in PBS at room temperature for 2 hours. Biotinylated human PVR as a ligand and the anti-human TIGIT antibody were mixed at various molar ratios of 1:0, 1:0.1, 1:1, and 1:10 and reacted at room temperature for 10 minutes. Then, 50 $\mu$l of the resulting reaction product was placed on a plate coated with human TIGIT and incubated at room temperature for 2 hours. The result was washed 3 times with PBST (0.05% Tween 20 in PBS), and 50 $\mu$l of anti-SA-HRP diluted 1:3000 with PBS was added thereto, followed by incubation at room temperature for 1 hour. The result was washed 3 times with PBST (0.05% Tween 20 in PBS) and 50 $\mu$l of a TMB solution was added thereto. The result was allowed to react at room temperature for 5 minutes, 50 $\mu$l of a TMB stop solution was added thereto, and the absorbance was measured at 450 nm in an ELISA leader.
The results are shown in FIG. 3. A blocking assay for TIGIT and PVR was performed on 11 types of anti-TIGIT antibodies, and the results of the assay showed that all 11 types of anti-TIGIT antibodies blocked the ligand.

**Example 4. Determination of affinity of anti-TIGIT antibodies**

4.1 Determination using Octet

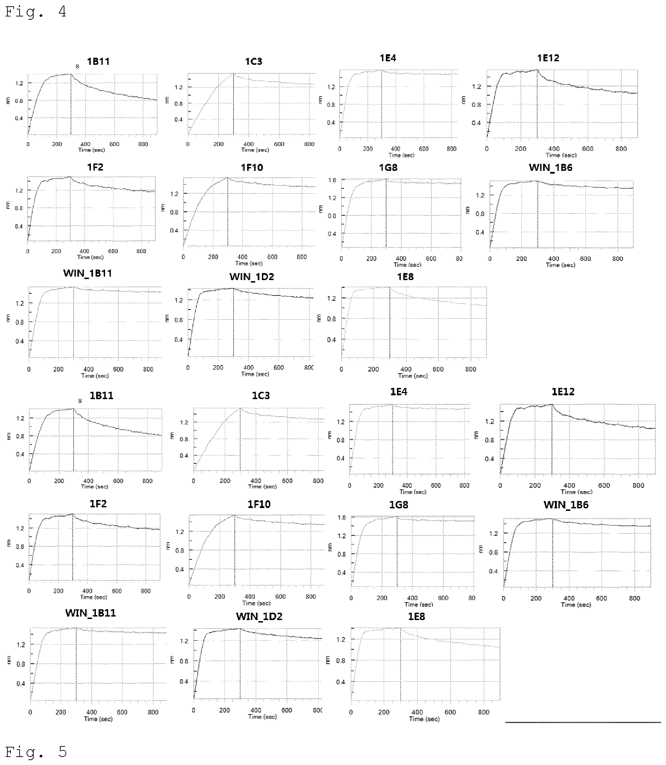

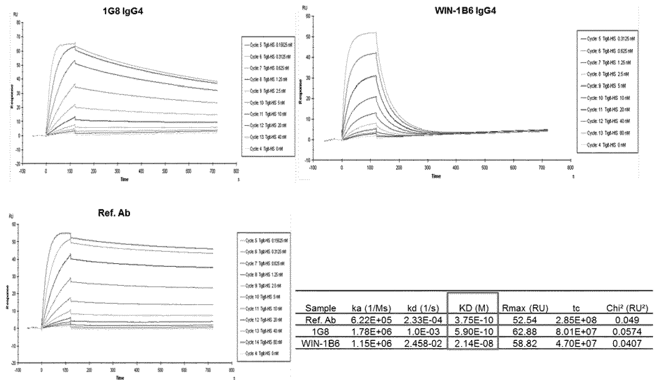

A 5 $\mu$g/ml of human TIGIT-HIS was prepared by diluting the same in a 1XKB buffer. Anti-human TIGIT antibodies were prepared through 1/2 serial dilution with 1XKB buffer from a starting concentration of 100 nM. A penta-His sensor was reacted with 1XKB buffer for 10 minutes, and the affinity of the antibody to human TIGIT-HIS was measured using Octet. The results are shown in Table 3 and FIG. 4.
[Table 3]

| Sample ID | KD (M) | Full R^2 | kon(1/Ms) | kdis(1/s) |
|---|---|---|---|---|
| WIN-1B11 | 3.34E-10 | 0.9806 | 3.89E+05 | 1.30E-04 |
| WIN-1B6 | 4.43E-10 | 0.9795 | 4.65E+05 | 2.06E-04 |
| 1G8 | 2.24E-10 | 0.9867 | 4.15E+05 | 9.32E-05 |
| 1F10 | 1.32E-09 | 0.9942 | 1.76E+05 | 2.32E-04 |
| 1F2 | 8.97E-10 | 0.9684 | 4.80E+05 | 4.31 E-04 |
| 1E12 | 1.47E-09 | 0.9401 | 4.99E+05 | 732E-4 |
| 1E4 | 2.23E-10 | 0.9792 | 4,54E+05 | 1.01E-04 |

(continued)

| Sample ID | KD (M) | Full R^2 | kon(1/Ms) | kdis(1/s) |
|---|---|---|---|---|
| 1C3 | 3.55E-09 | 0.9913 | 8.97E+04 | 3.18E-04 |
| 1E8 | 9.37E-10 | 0.9585 | 5.63E+05 | 5.28E-04 |
| WIN-1D2 | 5.71E-10 | 0.975 | 4.88E+05 | 2.78E-04 |
| 1B11 | 3.16E-09 | 0.9576 | 3.21 E+05 | 1.02E-03 |

The affinity of 11 anti-TIGIT antibodies was measured using Octet and the result showed that among them, 1B11, 1E12, 1F2, and 1E8 had low dissociation compared to other clones.

4.2 Conversion to IgG4-type anti-TIGIT antibodies

The heavy-chain variable region and light-chain variable region of each of the final five anti-human TIGIT antibodies were synthesized.

[Table 4]

| Clone name | Heavy chain variable region sequence | SEQ ID No. |
|---|---|---|
| 1F10 | EVQLVESGGGLVKPGGSLRISCAAS**GFTFSNYN**MIWVRQAPGKGLEWVSSI**SSSASYI**YYADSVKGRFTISRDNAKNSLYLQMNSLRAEDTAVYYCAR**DEGSRDS**WNNGPYYYSGMDVWGQGTTVTVSS | 28 |
| 1G8 | EVQLVQSGAEVKKPGASVKVSCKAS**GYTFTSYY**MHWVRQAPGQGLEWMGI**INPSGGST**SYAQKFQGRVTMTRDTSTSTVYMELSSLRSEDTAVYYC**ASRSGSG**WFGALDYWGQGTLVTVSS | 29 |
| WIN_1B 6 | QVQLVQSGAEVKKPGASVKVSCKAS**GYTFTSYG**ISWVRQAPGQGLEWMGW**ISAYNGNT**NYAQKLQGRVTMTTDTSTSTAYMELRSLRSDDTAVYYC**ARAG**WEQQLGFDYWGQGTLVTVSS | 30 |
| WIN_1D 2 | QVQLVQSGAEVKKPGASVKVSCKAS**GYTFTSYG**ISWVRQAPGQGLEWMGW**ISAYNGNT**NYAQKLQGRVTMTTDTSTSTAYMELRSLRSDDTAVYYC**ARAG**WEQQLGFDYWGQGTLVTVSS | 31 |
| 1E8 | QVQLVQSGTEVKKPGASVKVSCKAS**GYTFSSYA**ITWVRQAPGQGLEWMGW**ISAYNGNT**NYAQKLQGRVTMTTDTSTSTAYMELRSLRSDDTAVYYC**ARVDF**WSGYNYFDYWGQGTLVTVSS | 32 |
| Referen ce | EVQLQQSGPGLVKPSQTLSLTCAIS**GDSVSSNSA**AWNWIRQSPSRGLEWLGK**TYYRFKWYS**DYAVSVKGRITINPDTSKNQFSLQLNSVTPEDTAVFYCTR**ESTTY**DLLAGPFDYWGQGTLVTVSS | 33 |
| Clone name | Light chain variable region sequence | |
| 1F10 | AIQMTQSPSSLSASVGDRVTITCRAS**QSISRY**LNWYQHKPGKAPKLLIY**GASSLQS**GVPSRFRGSGSGTDFTLTISSLQPEDFATYYC**QQSYTTPGAFT**FGGGTKVEIKR | 34 |
| 1G8 | EIVLTQSPGTLSLSPGERATLSCRAS**QSVSSSY**LAWYQQKPGQAPRLLIY**GASSRAT**GIPDRFSGSGSGTDFTLTISRLEPEDFAVYYC**QQYGSSPGGT**FGQGTKVEIKR | 35 |
| WIN_1B 6 | AIQLTQSPSSLSASVGDRVTISCRAS**QTIRSY**LNWYQQKPGKAPKLLIY**AASSLQS**GVPLRFSGSGSGTDFTLTISSLQPEDFATYYC**QQSYSTLPLT**FGGGTKVEIKR | 36 |
| WIN_1D 2 | AIQLTQSPSSLSASVGDRVTITCRAS**QSISSY**LNWYQQKPGKAPKLLIY**AASSLQS**GVPSRFSGSGSGTDFTLTISSLQPEDFATYYC**QQSYSTLAIT**FGQGTRLEIKR | 37 |

(continued)

| Clone name | Light chain variable region sequence | |
|---|---|---|
| 1E8 | DIQMTQSPSSLSASVGDRVTITCRAS**QGISNY**LAWYQQKPGKVPKVLIY**AASTLQS**GV PSRFSGSGSGTDFTLTISSLQPEDVATYYC**QKSNSAPLT**FGGGTKVEIKR | 38 |
| Referen ce | DIVMTQSPDSLAVSLGERATINCKSSQTVLYS**SNNKKY**LAWYQQKPGQPPNLLIY**WA STRES**GVPDRFSGSGSGTDFTLTISSLQAEDVAVYYC**QQYYSTPFT**FGPGTKVEIKR | 39 |

[Table 5]

| Clone name | CDR1 | CDR2 | CDR3 |
|---|---|---|---|
| 1F10VH | GFTFSNYN (SEQ ID NO: 3) | SSSASYI (SEQ ID NO: 4) | DEGSRDS (SEQ ID NO: 5) |
| 1F10 VL | QSISRY (SEQ ID NO: 14) | GASSLQS (SEQ ID NO: 15) | QQSYTTPGAFT (SEQ ID NO: 16) |
| 1G8VH | GYTFTSYY (SEQ ID NO: 6) | INPSGGST (SEQ ID NO: 7) | ASRSGSG (SEQ ID NO: 8) |
| IG8 VL | QSVSSSY (SEQ ID NO: 17) | GASSRAT (SEQ ID NO: 18) | QQYGSSPGGT (SEQ ID NO: 19) |
| WiN_1B6 VH | GYTFTSYG (SEQ ID NO: 9) | ISAYNGNT (SEQ ID NO: 10) | ARAG (SEQ ID NO: 11) |
| WIN_1B6 VL | QTIRSY (SEQ ID NO: 20) | AASSLQS (SEQ ID NO: 21) | QQSYSTLPLT (SEQ ID NO: 22) |
| WIN_1D2 VH | GYTFTSYG (SEQ ID NO: 9) | ISAYNGNT (SEQ ID NO: 10) | ARAG (SEQ ID NO: 11) |
| WIN_1D2 VL | QSISSY (SEQ ID NO: 23) | AASSLQS (SEQ ID NO: 21) | QQSYSTLAIT (SEQ ID NO: 24) |
| LE8 VH | GYTFSSYA (SEQ ID NO: 12) | ISAYNGNT (SEQ ID NO: 10) | ARVDF (SEQ ID NO: 13) |
| IE8 VL | QGISNY (SEQ ID NO: 25) | AASTLQS (SEQ ID NO: 26) | QKSNSAPLT (SEQ ID NO: 27) |

4.3 Biacore experiment

(1) Immobilization

10 µg/ml of anti-protein A was prepared by diluting the same in an acetate 4.0 buffer. 100 nM NHS, 400 mM EDC and 1 M ethanolamine were prepared. The sensor chip used herein was a CM5. The contact time was set at 300s (5 min), the flow rate was set at 5 µg/ml, and the immobilization level was set at 2,500 RU. After performing running, whether the RU reached the target level was monitored in the system control window.

(2) KD measurement

The capture antibodies 1G8 and WIN_1B6 and a reference antibody were diluted to 1 µg/ml in 1XHBS-EP buffer, and the target level was set to 300 RU. The antigen TIGIT-HIS was subjected to 1/2 serial dilution from 160 nM with 1XHBS-EP and reacted for a set contact time of 120 s and a set dissociation time of 600 s. When running was completed, ka, kd, and KD were obtained by fitting using evaluation software.
The results are shown in FIG. 5. Affinities of 1G8, WIN_1B6, and the reference antibody were measured using BIACORE. The reference antibody and candidate antibody 1G8 exhibited affinity of nM or higher, whereas the other candidate antibody, WIN_1B6, exhibited affinity of 10 nM or higher.

**Example 5. Determination of binding capacity between TIGIT expressed on the cell surface and TIGIT candidate antibody**

In order to prepare a cell line stably expressing TIGIT, Jurkat cells (Jurkat E6.1 (ATCC; TIB-152TM) were transfected with TIGIT cDNA, treated with 1 mg/ml of antibiotic G418, and selected to prepare a TIGIT-overexpressing cell line (Jurkat-TIGIT). The Jurkat-TIGIT cell line was resuspended in DPBS supplemented with 2% (v/v) FBS (hereinafter referred to as "FACS buffer") and centrifuged at 1,500 rpm. The cells were resuspended in FACS buffer so that the number of cells was $3\times10^6$ cells/ml, and 100 µl of the suspension was added to each well of a U-bottom 96-well plate.

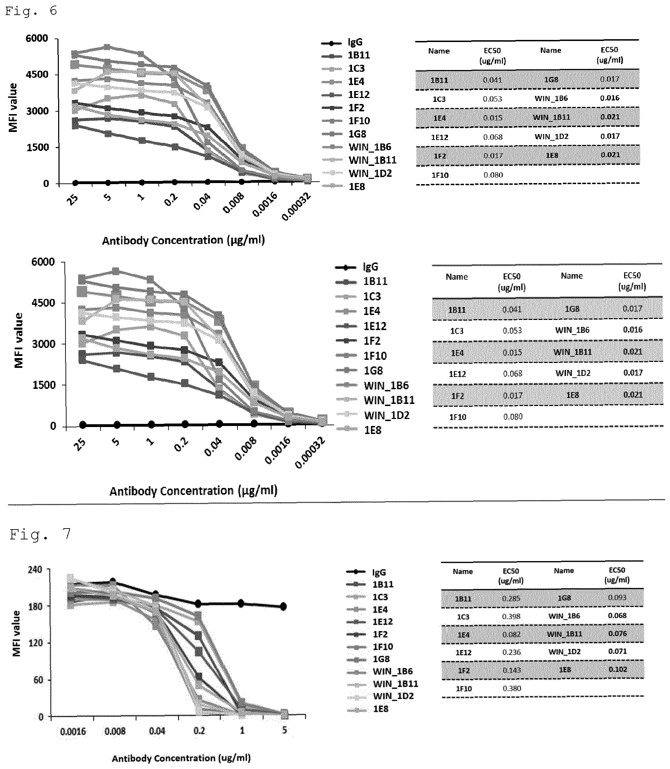

Then, the cells and culture solution of each well were recovered and centrifuged at 1,500 rpm, and then the supernatant was discarded. The recovered cells were resuspended in 50 μl of FACS buffer supplemented with 0.5 μl of a human Fc block (BD Pharmingen; Cat.# 564220) solution and incubated at 4°C for 15 minutes. The TIGIT candidate antibody or human IgG4 (Sigma; Cat.# 14639) was diluted in 50 μl of FACS buffer at the 2-time of the concentration 25 μg/ml, 5 μg/ml, 1 μg/ml, 0.2 μg/ml, 0.04 μg/ml, 0.008 μg/ml, 0.0016 μg/ml, or 0.00032 μg/ml. 50 μl of the previously diluted TIGIT candidate antibody or human IgG4 was added to the cells containing an Fc blocker such that the concentration was adjusted to each of 25 μg/ml, 5 μg/ml, 1 μg/ml, 0.2 μg/ml, 0.04 μg/ml, 0.008 μg/ml, 0.0016 μg/ml, and 0.00032 μg/ml and reacted at 4°C for 1 hour 30 minutes. The cells reacted with the TIGIT candidate antibody were resuspended in FACS buffer, and then centrifuged at 1,500 rpm and washed. This series of processes was repeated twice. Phycoerythrin (hereinafter, referred to as "PE")-conjugated goat anti-human Fab'2 (Sigma; Cat.# P8047) was diluted in FACS buffer at a volume ratio of 1:500, 100 μl thereof was added to each well, and the reaction was allowed to proceed in the dark at 4°C for 30 minutes. The cells reacted with PE were recovered, resuspended in FACS buffer, and centrifuged at 1,500 rpm, the supernatant was discarded, and the residue was washed. This series of processes was repeated twice. Then, the cells were resuspended in 100 μl of fixation buffer (BD Cytofix™; Cat.# 554655) and incubated in the dark at 4°C for 30 minutes. The cells reacted with the fixation buffer were recovered, resuspended in FACS buffer, and centrifuged at 1,500 rpm, the supernatant was discarded, and the residue was washed. This series of processes was repeated twice. The washed cells were resuspended in 200 μl of FACS buffer, and then the median fluorescence intensity (MFI) of PE with which the cells were labeled was compared using a FACS LSR-Fortessa instrument. All FACS analysis was performed using FlowJo software. The results are shown in FIG. 6. The results showed that WIN_1B6, WIN_1D2, 1E8, 1F10, and 1G8, among the TIGIT candidate antibodies, exhibited high binding capacity.

### Example 6. Determination of competitiveness of TIGIT candidate antibody for PVR, ligand of TIGIT

The TIGIT-overexpressing cell line (Jurkat-TIGIT) was resuspended in DPBS supplemented with 2% (v/v) FBS (hereinafter, FACS buffer), centrifuged at 1,500 rpm, and resuspended in FACS buffer so that the number of cells was $3 \times 10^6$ cells/ml, and 100 μl of the suspension was added to each well of a U-bottom 96-well plate. Then, the cells and culture solution of each well were recovered and centrifuged at 1,500 rpm, and then the supernatant was discarded. The recovered cells were resuspended in 50 μl of FACS buffer supplemented with 0.5 μl of a human Fc block (BD Pharmingen; Cat.# 564220) solution and incubated at 4°C for 15 minutes.

PVR-Fc purified from HEK293 cells (Thermo Fisher Scientific; Cat.# A14527) was added in an amount of 10 μg in 0.5 μl to each well and reacted at 4°C for 1 hour. The cells reacted with PVR-Fc were resuspended in FACS buffer, centrifuged at 1,500 rpm, and washed. This series of processes was repeated twice. TIGIT candidate antibody or human IgG4 (Sigma; Cat.# 14639) was diluted in 100 μl of FACS buffer at concentrations of 10 μg/ml, 0.2 μg/ml, 0.04 μg/ml, 0.008 μg/ml, 0.0016 μg/ml, and 0.00032 μg/ml, 100 μl thereof was added to each well, and the cells were resuspended and then reacted at 4°C for 1 hour. The cells reacted with the TIGIT candidate antibody were resuspended in FACS buffer, centrifuged at 1,500 rpm, and washed. This series of processes was repeated twice. Each well was treated with an anti-PVR-PE antibody (Invitrogen; Cat. # 12-1550-41) and allowed to react in the dark at 4°C for 30 minutes. The cells reacted with the anti-PVR-PE antibody were recovered, resuspended in FACS buffer, and centrifuged at 1,500 rpm, the supernatant was discarded and the residue was washed. This series of processes was repeated twice. Then, the cells were resuspended in 100 μl of fixation buffer (BD Cytofix™; Cat. # 554655) and incubated in the dark at 4°C for 30 minutes. The cells reacted with the fixation buffer were recovered, resuspended in FACS buffer, and centrifuged at 1,500 rpm, the supernatant was discarded, and the residue was washed. This series of processes was repeated twice. The washed cells were resuspended in 200 μl of FACS buffer. Then, the median fluorescence intensity (MFI) of PE with which the cells were labeled was compared using a FACS LSR-Fortessa instrument. All FACS analysis was performed using FlowJo software. The results are shown in FIG. 7. The results showed that among the TIGIT candidate antibodies, WIN_1B6, WUN_1B11, WIN_1D2, 1G8, and 1E8 strongly bound to PVR.

### Example 7. Analysis of T-cell activation due to inhibitory activity of TIGIT candidate antibody on binding between TIGIT and PVR

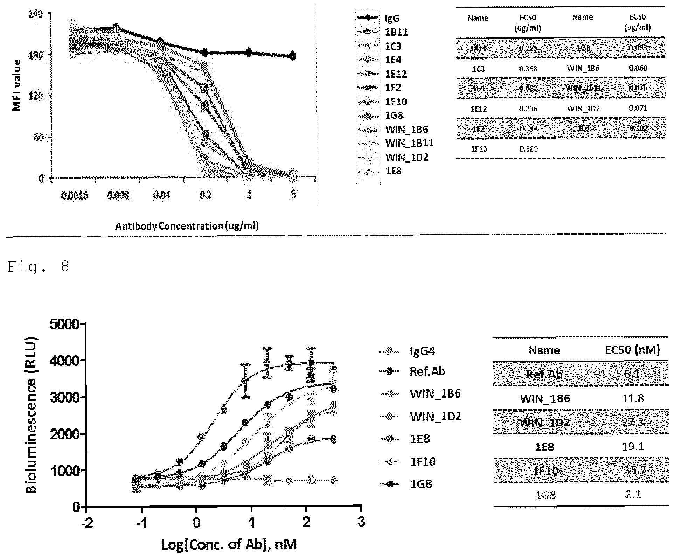

T-cell activation by the TIGIT candidate antibody was detected using the TIGIT/CD155 blockade bioassay kit (Promega, Cat No. J2305). One vial of thaw-and-use TIGIT effector cells stored at -140°C was thawed and resuspended in 12 ml of RPMI1640 (Gibco, Cat No. 11875-065) supplemented with 10% (v/v) FBS. 80 μl of the cell solution was added into the inner 60 wells of a white 96-well flat-bottom assay plate and incubated at 37°C for 16 to 18 hours. The next day, one vial of thaw-and-use CD155 aAPC/CHO-K1 cells stored at -140°C was thawed and resuspended in 3 ml of RPMI1640 (Gibco, Cat No. 11875-065) supplemented with 10% (v/v) FBS. The TIGIT candidate antibody was prepared at the highest concentration of 300 μg/ml (6X), and was serially diluted 10 times by 2.5x each time. 20 μl of an antibody was added to the TIGIT effector cell plate incubated the previous day, 20 μl of the thaw-and-use CD155 aAPC/CHO-K1 cell suspension was added thereto, and the result was incubated for 6 hours at 37°C in the presence of $CO_2$ in an incubator. After 6 hours, the 96-well plate was taken out from the incubator and allowed to stand at room temperature for 20 minutes. A Bio-Glo substrate was mixed with 10 ml of buffer, and each of the 60 wells containing the cell suspension was treated with 120 μl of the resulting mixture and allowed to stand for 10 minutes. Luminescence was measured using the GloMax Discover System. The results are shown in FIG. 8. The results showed that among the five TIGIT candidate antibodies, upon treatment with 1G8, $EC_{50}$ was 2.1 nM, which demonstrated that among the candidate antibodies, 1G8 exhibited the highest ability to activate T cells.

**Example 8. Determination of cross-reaction of TIGIT candidate antibody**

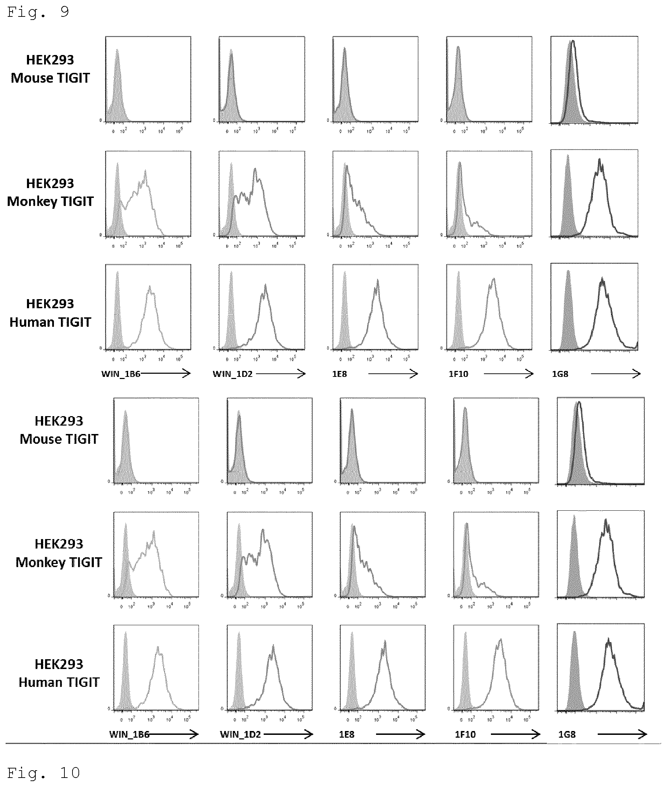

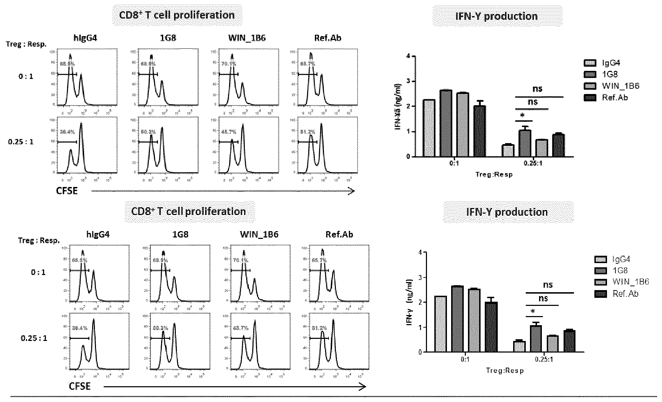

In order to determine whether or not the TIGIT antibody has binding ability to TIGIT of mice, monkeys and humans, each of pEF1a-AcGFP-N1 vector (Clontech, Cat No. 631973) as a control vector, mouse TIGIT, monkey TIGIT, and human TIGIT plasmid was added in an amount of 20 μg to HEK293 cells and then transfected with Lipofectamine™ 3000 (Invitrogen, Cat No. L3000001). After transfection for 48 hours, GFP expression was observed using a fluorescence microscope, and the cells were detached by treatment with 1 ml of TrypLE Express Solution (Thermo Fisher Scientific; Cat.#12605010), and then diluted in 9 ml of DMEM (Gibco, Gibco, Cat No. 11995-065) supplemented with 10% (v/v) FBS, and centrifuged at 1,200 rpm for 5 minutes, and the supernatant was removed. The result was washed once with PBS, and then the cells were resuspended in FACS buffer to a concentration of $5\times10^5$ cells/100 μl. 1 μl of a human Fc block solution was added to each sample and reacted at 4°C for 10 minutes. The sample was treated at a concentration of 1 μg/$5\times10^5$ cells with human IgG4, WIN_1B6, WIN_1D2, E8, 1F10, and 1G8, and reacted at 4°C for 1 hour. The result was washed with FACS buffer, PE-labeled goat-anti-human Fab'2 antibody was diluted at 1:200 in FACS buffer, 100 μl of the dilution was added to each sample, and reaction was allowed to proceed at 4°C for 30 minutes. The result was centrifuged in 200 μl of FACS buffer at 1,200 rpm for 5 minutes to remove the supernatant. The cells were resuspended in 300 μl of fixation buffer. The cells expressing GFP were screened using a FACS LSR-Fortessa instrument, and TIGIT candidate antibodies were identified using a PE fluorescence channel and analyzed using FlowJo software. The results are shown in FIG. 9. The results showed that, among the TIGIT candidate antibodies, WIN_1B6, WIN_1D2, E8, and 1G8 bound to TIGIT of humans as well as monkeys based on binding ability to proteins expressed on the cell surface. However, the results showed that binding to TIGIT did not occur in the case of mice.

**Example 9. Determination of inhibitory activity of TIGIT candidate antibody on regulatory T cells**

9.1 Isolation of mononuclear cells from peripheral blood (PBMC)

Blood was transferred from a concentrated red blood cell bag (200-250 mL) to a disposable bottle using an 18G needle and a 50 mL syringe, and was diluted at a ratio of 1:1 in DPBS. 15 mL of Ficoll-Paque medium (GE Healthcare, Cat No. 17-1440-03) was prepared in a fresh 50 mL conical tube, and 30 mL of diluted blood was slowly layered on the Ficoll-Paque medium by placing a pipette tip on the wall of the conical tube. The result was centrifuged at 1,500 rpm and 25°C for 30 minutes (no brake). After centrifugation, the PBMC layer was carefully collected and transferred to a 50 ml fresh conical tube, a washing buffer was added to adjust a total volume to 50 ml, and centrifugation was performed at 1,500 rpm at 4°C for 10 minutes. The supernatant was discarded, the cells were resuspended in a washing buffer and centrifuged at 1,500 rpm and 4°C for 10 minutes, and the supernatant was discarded. Then, the result was washed with IMDM (GIBCO, Cat No. 12440-) supplemented with 10% (v/v) FBS 053), the number of cells and viability thereof were determined, and the cells were incubated on a 6-well plate for 16 to 18 hours.

9.2 Isolation of Regulatory T cell

A regulatory T cell isolation kit (STEMCELL, Cat No. 18063) was used to isolate regulatory T cells from PBMCs incubated the day before. $5\times10^8$ cells/ml of isolated human PBMCs were placed in a round-bottom tube, 100 μl of a CD25 positive selection cocktail was added thereto, and then reaction was allowed to proceed at room temperature for 5 minutes. 60 μl of releasable RapidSpheres (vortexed before use) was added thereto, 100 μl of a CD4$^+$ T cell enrichment cocktail was further added thereto, and then reaction was allowed to proceed at room temperature for 5 minutes. After resuspension, the round-bottom tube was inserted into the magnet and reacted at room temperature for 10 minutes. Then, the supernatant (CD4$^+$ CD25$^-$) was carefully removed with a pipette and transferred to a fresh tube. The tube was carefully separated from the magnet, 2.5 ml of EasySep buffer was added thereto, and the result was carefully resuspended with a pipette 2-3 times, placed on the magnet again, and reacted at room temperature for 5 minutes, and then the supernatant (CD4$^+$ CD25$^-$) was carefully removed with a pipette. This series of processes was repeated twice. The tube was separated from the magnet and EasySep buffer was added thereto in the same amount as the initial amount, resuspension was performed 5 times or more with 200 μl of release buffer, 100 μl of a CD127$^{high}$ depletion cocktail was added thereto, and then the reaction was allowed to proceed at room temperature for 5 minutes. 20 μl of Dextran RapidSpheres (vortexed before use) were added thereto, followed by reaction at room temperature for 5 minutes and resuspension. Then, a round-bottom tube was inserted into a magnet, followed by reaction at room temperature for 5 minutes. The supernatant (CD4$^+$CD25$^+$CD127$^{low}$) was carefully decanted with a pipette, transferred to a fresh tube, and washed once with IMDM (GIBCO, Cat No. 12440-053) supplemented with 10% (v/v) FBS. The number of cells was determined.

9.3 Labeling responder cells with CFSE

1 × 10$^6$ cells/ml (1 ml) of the PBMCs incubated the day before were placed in a 15 ml tube, 1 ml of 20 μM CFSE (eBioscience, Cat No. 65-0850-84) was added thereto, the cells were mixed thoroughly, entry of light was blocked with silver foil, and a reaction was allowed to proceed in a 37°C CO$_2$ incubator for 20 minutes. After 20 minutes, cold IMDM supplemented with 10 ml of 10% (v/v) FBS was added thereto, followed by centrifugation at 1,200 rpm and 4°C for 5 minutes, and this process was repeated twice.

9.4 Co-culture of regulatory T cells with CFSE-labeled responder cells

The CFSE-labeled responder cells were resuspended in IMDM supplemented with 10% (v/v) FBS and seeded at 2 × 10$^5$ cells/100 μl on a 96-well round plate, and regulatory T cells were added thereto at ratios of 0:1, 0.1:1 (2×10$^4$), 0.25:1 (5×10$^4$), and 0.5:1 (1×10$^5$) (Treg: responder). Soluble anti-CD3/anti-CD28 (2 μg/ml) was added to each well and human IgG4 and TIGIT antibody were added at a concentration of 10 μg/ml to each well, followed by incubation for 5 days in a 37°C 5% CO$_2$ incubator. After 5 days, the cells and culture medium were recovered, centrifuged at 1,500 rpm, the supernatant was stored at -80°C for IFN-γ ELISA, the remaining cells were recovered, and Fc receptors were blocked at 4°C in fresh FACS buffer for 15 minutes. The plate was treated with antibodies to CD45-PE (TONBO, Cat No. 50-0459-T100), CD3-Percific blue (Biolegend, Cat No. 300330), CD8-APC (TONBO, Cat No. 20-0088-T100), CD4-PE-Cy7 (TONBO, Cat No. 60-0049-T100), and CD25-APC-Cy7 (Biolegend, Cat No. 302614), each being marked with a different dye, followed by reaction at 4°C for 30 minutes. After the reaction, a FACS buffer was added and centrifugation was performed. This process was repeated three times. The cells were resuspended in 100 μl of fixation buffer (BD Cytofix; cat. #554655) and reacted at 4°C for 30 minutes. The result was washed twice with FACS buffer and resuspended in 200 μl of FACS buffer for assay. Cell proliferation was determined based on CFSE in CD8 cells using a FACS LSR-Fortessa instrument and analyzed using FlowJo software. The results are shown in FIG. 10. The results showed that the function of regulatory T cells was suppressed by the TIGIT candidate antibody and that the number of CD8 T cells was increased by 14% compared to the control group.

9.5 Detection of IFN-γ secretion by TIGIT candidate antibody

The sample supernatant stored at -80°C was thawed at room temperature and centrifuged at 1,500 rpm for 5 minutes. To measure the amount of secreted IFN-γ, a human IFN-γ immunoassay kit (R&D SYSTEMS, Cat No. DIF50) was used. All reagents used for ELISA were prepared at room temperature. As many microplate strips as the number of samples were prepared, and 100 μl of diluent RD1-51 was added to each well to which the sample would be fed. The supernatant was diluted 20x with calibrator diluent RD6-21. IFN-γ standard stock (1,000 pg/ml) was diluted to 500 pg/ml, and was then serially diluted 6 times at a volume ratio of 1:1. 100 μl of a standard and 100 μl of a sample were added to each well, followed by reaction at room temperature for 2 hours. The result was washed 3 times with wash buffer, and each well was treated with 200 μl of a human IFN-γ conjugate, followed by reaction at room temperature for 2 hours. The result was washed 3 times with wash buffer, and each well was treated with 200 μl of a substrate solution, followed by reaction at room temperature for 30 minutes. Each well was treated with 50 μl of a stop solution, and the O.D. value was measured at a wavelength of 540 nm or 570 nm using molecular dynamics reader equipment. The measured values were analyzed using SoftMax Pro 5.4.1 program. The results are shown in FIG. 10. The results showed that the function of regulatory T cells was inhibited by the TIGIT candidate antibody and thus the amount of secreted IFN-γ, measured in the culture supernatant, was about two times higher than in the control group.

**Example 10. Determination of anticancer activity of TIGIT candidate antibody**

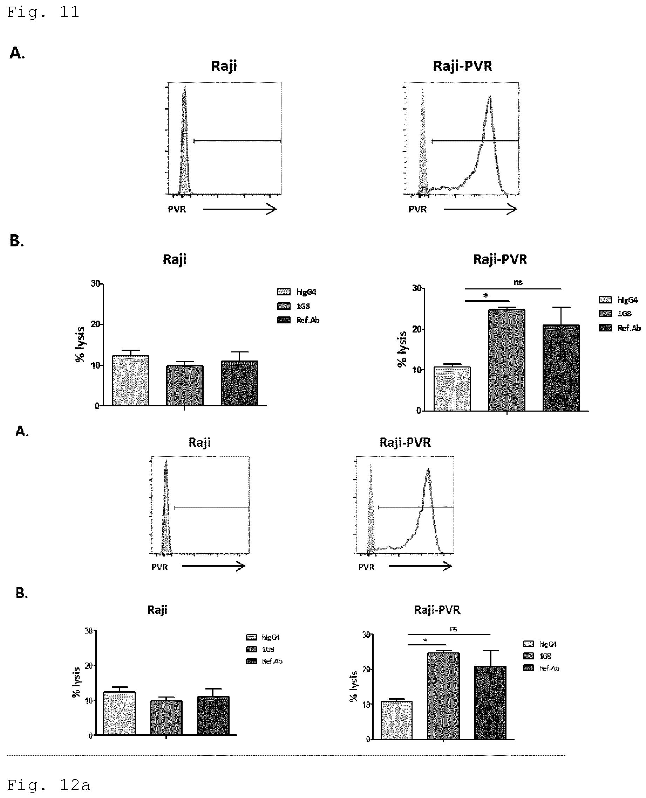

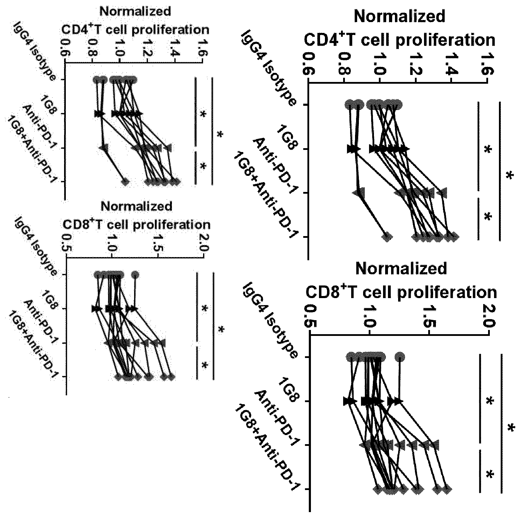

Raji cancer cells and the Raji-PVR cell line prepared to overexpress PVR were resuspended at a concentration of 1×10$^5$ cells/100 μl in RPMI 1640 medium (Thermo Fisher Scientific; Cat.# 11875093) in a 15 mL tube, 30 μℓ of calcein-AM (final 30 μM, Invitrogen; Cat.#C3099) was added thereto, followed by reaction in the dark at 37°C for 1 hour. After 1 hour, the result was washed twice with RPMI 1640 medium, resuspended at a concentration of 1×10$^5$ cells/100 μℓ in RPMI 1640 medium, and then added to a 96-well-plate. The expanded NK cells were added to each well along with cancer cells at a ratio of 3:1 thereto, and at this time, 1G8 was added to each well at a concentration of 10 $\mu$g/ml, and the result was treated with human IgG4 as a control group in the same manner as above and co-cultured in 5% $CO_2$ in the dark at 37°C for 6 hours. After 6 hours, the 96-well-plate was centrifuged at 2,000 rpm for 3 minutes, 100 $\mu$l of the supernatant was collected and transferred to a 96-well-black plate, and the value of each sample was measured at wavelengths of 485 nm/535 nm using a multiple reader. The lysis value was assayed using the following formula. The results are shown in FIG. 11. The results showed that cancer cell apoptosis by NK cells was increased in PVR-overexpressing cells on treatment with 1G8, and this is due to specific targeting for TIGIT in a PVR-expression-dependent manner.

$$\text{Lysis} = \frac{\text{Sample} - \text{Spon}}{\text{Max} + (\text{MM} - \text{MT}) - \text{Spon}} \times 100$$

**Example 11. Determination of increase in T-cell reactivity upon treatment with combination of TIGIT candidate antibody (1G8) with PD-1 antibody**

11.1. Differentiation into mature dendritic cells after isolation of CD14-positive cells from mononuclear cells isolated from peripheral blood (PBMC)

Cryopreserved healthy donor-derived peripheral blood mononuclear cells (PBMCs) were rapidly thawed in a 37°C water bath and transferred to a 50 mL conical tube, and a thawing medium (RPMI, Gibco 11875-093 + 10% FBS, Gibco 16000-044) was added dropwise thereto while shaking the same. Then, the supernatant was removed by centrifugation at 1200 rpm at 4°C for 10 minutes, the residue was resuspended in 40 mL of MACS buffer (PBS+0.5% FBS+2mM EDTA), and the number of cells was counted. The cells were treated at a concentration of 20 $\mu$L/$10^7$ cells with CD14 microbeads (Miltenyi Biotec, 130-050-201), treated with 80 $\mu$L of MACS buffer, and then incubated in the dark at 4°C for 15 minutes. Then, the supernatant was removed by centrifugation at 1,350 rpm at 4°C for 8 minutes, and the residue was resuspended in 500 $\mu$L of MACS buffer and then loaded on an LS column (Miltenyi Biotec 130-042-401) mounted on a QuadroMACS separator (Miltenyi Biotec, 130-090-976). The LS column was washed 3 times with 3 mL of MACS buffer, removed from the QuadroMACS separator, transferred to a 15 mL conical tube, and pressed with a plunger to obtain CD14-positive cells. The obtained CD14-positive cells were resuspended at a concentration of 1.2×$10^6$ cells/mL in complete RPMI (RPMI, Gibco A10491-01+10% FBS+55 $\mu$M $\beta$-Mercaptoethanol, Gibco 21985-023+1X Antibiotic-Antimycotic, Gibco 15240) and mixed with complete RPMI containing 2X GM-CSF (2×$10^3$ U/mL) (R&D systems, 215-GM-010) and 2X IL-4 (2×$10^3$ U/mL) (R&D systems, 204-IL-010) cytokines at a ratio of 1:1, and the result was added to a 6-well plate and then incubated in a $CO_2$ incubator at 37°C. After 3 days, 1.5 mL of the supernatant culture medium was removed from each well using a 1 mL pipette, and 2 mL of complete RPMI medium containing 1X GM-CSF+1X IL-4 was added to each well. After 2 days, 2 mL of the culture solution was removed from each of the 6 wells and 3 mL of the medium containing each cytokine GM-CSF (1000 U/mL)+IL-4 (1000 U/mL)+TNF-$\alpha$ (10 ng/mL) (R&D systems, 210-TA-010)+IL-1$\beta$ (10 ng/mL) (R&D systems, 201-LB-005)+IL-6 (10 ng/mL) (R&D systems, 206-IL-010)+PGE2 (1 $\mu$g/mL) (Sigma, P0409-1MG) was added thereto and the cells were incubated in a $CO_2$ incubator at 37°C for 2 days. On the 7th day after releasing and incubating the cells, mature dendritic cells adhered to the bottom were pipetted and collected in a 50 mL conical tube, the number of cells was counted, and the concentration thereof was adjusted to 1×$10^5$ cells/mL.

11.2. Isolation of pan T cells from mononuclear cells isolated from peripheral blood (PBMC) and VPD450 staining

Cryopreserved peripheral blood mononuclear cells (PBMCs) isolated from an allogeneic donor different from the donor from whom dendritic cells were isolated were rapidly thawed in a 37°C water bath and transferred to a 50 mL conical tube, and the thawed medium was added dropwise during mixing while shaking. Then, the result was centrifuged at 1,200 rpm at 4°C for 10 minutes to remove the supernatant and resuspended in 40 mL of MACS buffer, and the number of cells was counted. The cells were treated at a density of 10 $\mu$L/$10^7$ cells with pan T cell biotin antibodies (Miltenyi Biotec, 130-096-535), treated with 40 $\mu$L of MACS buffer, and incubated in the dark at 4°C for 5 minutes. Then, the cells were treated at a density of 20 $\mu$L/$10^7$ cells with anti-biotin microbeads (Miltenyi Biotec, 130-096-535), treated with 30 $\mu$L of MACS buffer, and incubated in the dark at 4°C for 10 minutes. After incubation, the final volume was adjusted to 500 $\mu$L with MACS buffer and then the resulting cell solution was loaded onto the LS column mounted on the QuadroMACS separator. The MACS buffer was loaded in an amount of 3 mL three times to each LS column, all of the cells released from the LS column were collected in a 15 mL conical tube, and the number of cells was counted. The collected T cells were centrifuged at 1,200 rpm at 4°C for 10 minutes, the supernatant was removed, and then the residue was resuspended in 1X PBS solution to adjust the final concentration to $2\times10^7$ cells/mL. A 1 $\mu$M VPD450 solution having the same volume as the solution containing the cells was thoroughly mixed with the cells, light was blocked with aluminum foil, and the reaction was allowed to proceed in a $CO_2$ incubator at 37°C for 20 minutes. Then, 10 mL of thawing medium was added, followed by centrifugation at 1,200 rpm at 4°C for 5 minutes, and the supernatant was removed. This series of processes was repeated twice. The resulting T cells were resuspended in complete RPMI medium to adjust the final concentration to $2\times10^6$ cells/mL.

11.3. Mixed lymphocyte reaction (MLR) test using dendritic cells and allogeneic T cells

The amount of complete RPMI medium was adjusted to co-culture the dendritic cells matured *in vitro* for 7 days obtained in 11.1 and the allogeneic T cells stained with VPD450 isolated in 2.2 at a ratio of 1:20, and the final concentration was adjusted as follows (dendritic cell final concentration: $1\times10^5$ cells/mL; final T-cell concentration: $2\times10^6$ cells/mL). $5\times10^3$ cells/50 $\mu$L of dendritic cells and $1\times10^5$ cells/50 $\mu$L of T cells were transferred by pipetting the same into each well of a 96-well U-bottom plate. The cells were transferred at a constant density and co-cultured for 5 days under the five following conditions: 1) only dendritic cells and T cells were cultured, 2) treatment with human IgG4 type isotype antibody (BioLegend, 403702), in addition to condition #1, 3) treatment with TIGIT candidate antibody (1G8) alone, in addition to condition #1, 4) treatment with PD-1 antibody (MSD, Pembrolizumab) alone, in addition to condition #1, and 5) treatment with a combination of candidate antibody (1G8) and PD-1 antibody TIGIT, in addition to condition #1. In all experiments, the final concentration of each antibody was adjusted to be 5 $\mu$g/mL. After treatment with each antibody, the 96-well plate was incubated in a $CO_2$ incubator at 37°C for 5 days. After 5 days, the cells and culture solution were recovered and centrifuged at 2,000 rpm, and the supernatant was stored in a -80°C freezer for human IFN-$\gamma$ ELISA. FACS buffer (1% FBS/sheath buffer) was added to the cells remaining in the 96-well plate, the result was centrifuged at 1,200 rpm and 4°C for 5 minutes, and the supernatant was removed. This series of processes was repeated twice. The residue was resuspended in 100 $\mu$L of FACS buffer, transferred to a 5 mL tube, treated with antibodies, and incubated in the dark at 4°C for 30 minutes. The antibodies used herein were FITC anti-CD8 (BioLegend 301006), PE anti-TIGIT (eBiosciences, 12-9500-42), PE-Cy7 anti-PD-1 (eBiosciences, 25-2799-42), APC anti-CD4 (Tonbo 20-0049-T100), APC-Cy7 anti-CD3 (BD 560176), and PerCP Cy5.5 anti-7AAD (BD 559925). Then, 1 mL of FACS buffer was added to each sample, followed by centrifugation at 2,000 rpm at 4°C for 3 minutes, and then the supernatant was removed to thereby obtain a sample. The cell proliferation level of each group was determined and analyzed through the VPD450 negative/positive proportions in CD4-positive T cells and CD8-positive T cells using LSR Fortessa. The results are shown in FIG. 12a.

As can be seen from FIG. 12a, the TIGIT candidate antibody (1G8)-treated group did not exhibit a significant increase in cell proliferation in both CD4-positive T cells and CD8-positive T cells compared to the IgG4 isotype antibody-treated control group, whereas the PD-1 antibody-treated group exhibited a statistically significant increase in cell proliferation. At this time, statistically significant, further increased cell proliferation was observed upon treatment with a combination of the PD-1 antibody and the TIGIT candidate antibody (1G8), compared to treatment with the PD-1 antibody alone.

11.4. Observation of change in IFN-$\gamma$ secretion

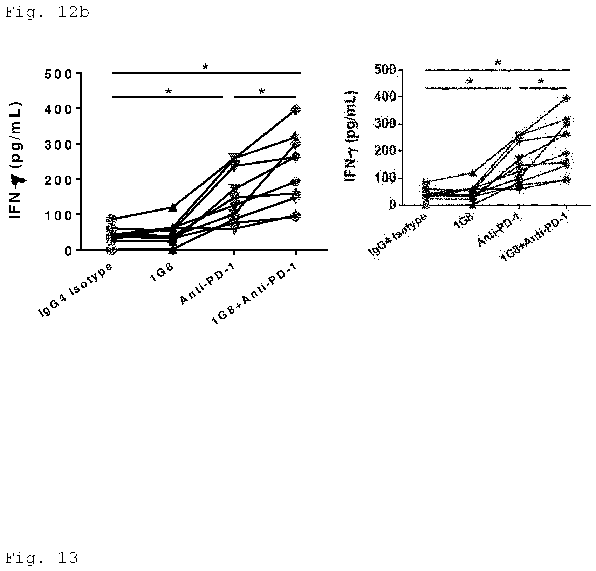

The sample supernatant stored at -80°C was thawed at room temperature and centrifuged at 1,500 rpm for 5 minutes. Human IFN-gamma Quantikine ELISA Kit (R&D SYSTEMS, DIF50) was used to measure the amount of IFN-$\gamma$ that was secreted. All reagents used for ELISA were prepared at room temperature. As many microplate strips as the number of samples were prepared, and 100 $\mu$l of diluent RD1-51 was added to each well to which the sample would be fed. The supernatant was diluted 20x with calibrator diluent RD6-21. IFN-$\gamma$ standard stock (1,000 pg/ml) was diluted to 500 pg/ml, and was then serially diluted 6 times at a volume ratio of 1:1. 100 $\mu$l of a standard and 100 $\mu$l of a sample were added to each well, followed by reaction at room temperature for 2 hours. The result was washed 3 times with wash buffer, and each well was treated with 200 $\mu$l of a human IFN-$\gamma$ conjugate, followed by reaction at room temperature for 2 hours. The result was washed 3 times with wash buffer, and each well was treated with 200 $\mu$l of a substrate solution, followed by reaction at room temperature for 30 minutes. Each well was treated with 50 $\mu$l of the stop solution, and the O.D. value was measured at a wavelength of 540 nm or 570 nm using molecular dynamics reader equipment. The measured values were analyzed using SoftMax Pro 5.4.1 program. The results are shown in FIG. 12b.

As can be seen from FIG. 12b, there was no difference in the amount of IFN-$\gamma$ secretion between the TIGIT candidate antibody-treated group and the control group, namely, the IgG4 isotype antibody-treated group, whereas there was a statistically significant increase in the amount of IFN-$\gamma$ secretion between the PD-1 antibody-treated group and the control group. At this time, statistically significant, further increased (47% in average) IFN-$\gamma$ secretion was observed upon treatment with a combination of the PD-1 antibody with the TIGIT candidate antibody (1G8), compared to treatment with the PD-1 antibody alone.

**Example 12. Determination of reduction of regulatory T cell activation marker expression by TIGIT candidate antibody (1G8)**

12.1. Treatment of mononuclear cells isolated from peripheral blood (PBMCs) of healthy donor with TIGIT candidate antibody (1G8)

Cryopreserved healthy donor-derived peripheral blood mononuclear cells were rapidly thawed in a 37°C water bath, then transferred to a 50 mL conical tube, and the thawing medium was added dropwise by mixing while shaking. Then, the result was centrifuged at 1,200 rpm at 4°C for 10 minutes to remove the supernatant, the cells were resuspended in 15 mL of a thawing medium, and the number of cells was counted. The cells were resuspended at $1 \times 10^7$ cells/mL in complete RPMI, $1 \times 10^6$ cells and 2 $\mu$L of Dynabeads human T-activator CD3/CD28 (gibco, 111.31D) were seeded to a total of 200 $\mu$L/well in 3 times on a 96-well U-bottom plate. Each group was treated with the corresponding antibody (1G8 or hIgG4) at a final concentration of 1 mg/mL and incubated for 6 days.

12.2. Analysis of transformation of regulatory T cells

The plate containing the cells incubated for 6 days was centrifuged at 2,000 rpm and at 4°C for 3 minutes to remove the supernatant, resuspended in 200 $\mu$L of PBS per well, and centrifuged at 2,000 rpm and at 4°C for 3 minutes. After removing the supernatant, in order to isolate dead cells, the residue was treated with LIVE/DEAD™ Fixable Aqua Dead Cell Stain Kit (Invitrogen, L34957) and incubated in the dark at 4°C for 30 minutes. Each well was further treated with 100 $\mu$L of FACS buffer and centrifuged at 2,000 rpm at 4°C for 3 minutes. The supernatant was removed and the residue was treated with antibodies and incubated in the dark at 4°C for 3 minutes for 30 minutes. The antibodies herein used were BV421 anti-CD25 (BD 562442), FITC anti-CD127 (BD 564423), BB700 anti-CD8 (BD 566452), R700 anti-CD4 (BD 564975), APC-H7 anti-CD3 (BD 560176), PE-Cy7 anti-CD39 (eBioscience 25-0399-41), PE-Cy7 anti-PD-1 (Biolegend 135216), Alexa647 anti-ICOS (Biolegend 313516), Alexa647 anti-TIGIT (Biolegend 372724), PE-Cy7 anti-LAG-3 (Biolegend 369310), PE-Cy7 anti-HLA-DR (BD 565096), and APC anti-CCM1 (R&D FAB2244A) . Each well was further treated with 100 $\mu$L of FACS and centrifuged at 2,000 rpm at 4°C for 3 minutes. The supernatant was removed and intracellular staining was performed in accordance with the protocol of FoxP3/transcription factor staining buffer set (eBioscience, 00-5523-00) . The antibodies used herein were PE anti-FoxP3 (eBioscience 12-4776-42) and APC anti-CTLA-4 (R&D FAB386A). The results of an assay of stained cells using LSR Fortessa are shown in FIG. 13.
As can be seen from FIG. 13, among the activation markers of regulatory T cells of a normal subject, the 1G8 antibody did not affect the expression of ICOS, PD-1, or LAG3, but significantly reduced the expression of CD39 and CTLA-4.

**Example 13. Analysis of exhaustion maker expression of CD8 T cells and regulatory T cells in patients with multiple myeloma**

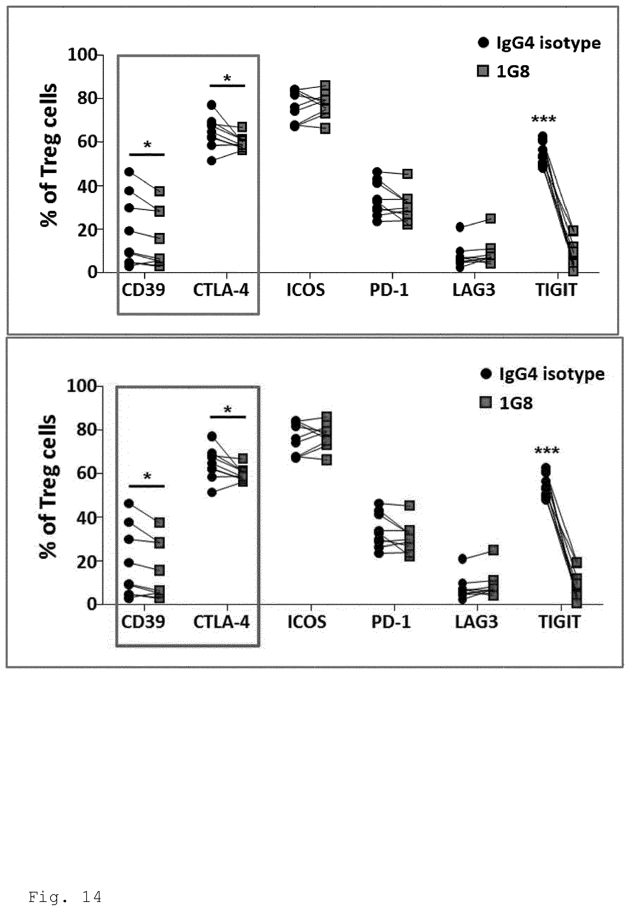

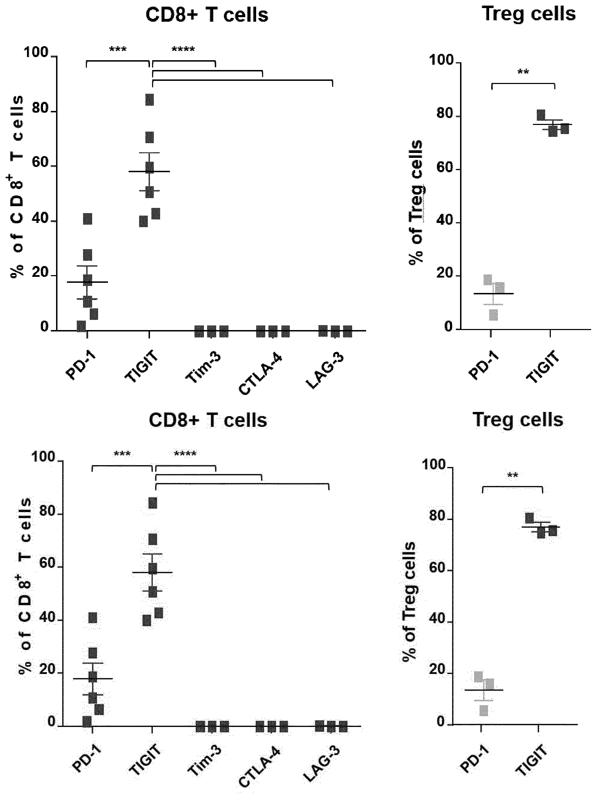

Cryopreserved peripheral blood mononuclear cells derived from multiple myeloma patients were rapidly thawed in a 37°C water bath, transferred to a 50 mL conical tube, and mixed dropwise with the thawing medium while shaking. Then, the cells were centrifuged at 1,200 rpm at 4°C for 10 minutes, the supernatant was removed, the cells were resuspended in 15 mL of a thawing medium, and the number of cells was counted. The thawed peripheral blood mononuclear cells were aliquoted into four FACS tubes, 2 mL of PBS was added thereto, and the result was centrifuged at 4°C at 2,000 rpm for 3 minutes. The supernatant was removed, and in order to isolate dead cells, the residue was treated with LIVE/DEAD™ Fixable Aqua Dead Cell Stain Kit and incubated at 4°C in the dark for 30 minutes. Each tube was further treated with 2 mL of FACS buffer and centrifuged at 4°C at 2,000 rpm for 3 minutes. After removing the supernatant and treating with antibodies, the cells were incubated in the dark for 30 minutes at 4°C. The antibodies used herein were Alexa700 anti-CD3 (BD 557943), PerCP-Cy5.5 anti-CD8 (Biolegend 344710), BB700 anti-CD8 (BD 566452), R700 anti-CD4 (BD 564975), BV421 anti-CD25 (BD 562442), BV786 anti-CD127 (BD 563324), FITC anti-CD138 (Biolegend 356508), PE-Cy7 anti-PD-1 (Biolegend 135216), BV421 anti-TIGIT (BD 747844), Alx647 anti-TIGIT (Biolegend 372724), APC anti-Tim-3 (R&D FAB2356aA), PE-Cy7 anti-LAG-3 (Biolegend 369310), and APC anti-CTLA-4 (R&D FAB386A). After staining, 2 mL of FACS buffer was added to each tube, and the cells were centrifuged at 4°C at 2,000 rpm for 3 minutes. The supernatant was removed and intracellular staining was performed in accordance with the protocol of the FoxP3/transcription factor staining buffer set. The antibodies used herein were PE anti-FoxP3 (eBioscience 12-4776-42) and APC anti-CTLA-4 (R&D FAB386A). The results of analysis of stained cells with LSR Fortessa are shown in FIG. 14.
As can be seen from FIG. 14, the expression of TIGIT among the five exhaustion markers of CD8 T cells in patients with multiple myeloma was significantly higher than that of the other four types. TIGIT was expressed in higher levels in regulatory T cells from multiple myeloma patients, compared to PD-1.

**Example 14. Determination of IFN-γ expression increase in peripheral blood mononuclear cells of multiple myeloma patients by TIGIT candidate antibody (1G8)**

14.1 Treatment of peripheral blood mononuclear cells of multiple myeloma patients with TIGIT candidate antibody (1G8)

Cryopreserved peripheral blood mononuclear cells derived from multiple myeloma patients were rapidly thawed in a 37°C water bath and transferred to a 50 mL conical tube, and a thawing medium was added dropwise by mixing while shaking. Then, the cells were centrifuged at 1,200 rpm at 4°C for 10 minutes, the supernatant was removed, the cells were resuspended in 15 mL of a thawing medium, and the number of cells was counted. The cells were resuspended at $5 \times 10^6$ cells/mL in complete RPMI, $1 \times 10^5$ cells/well and 2 μL of Dynabeads human T-activator CD3/CD28 were seeded 3 times on a 96-well U-bottom plate. Each group was treated at 1 mg/mL with the corresponding antibodies (TIGIT candidate antibody (1G8), PD-1 antibody, hIgG4, a combination of TIGIT candidate antibody (1G8) and PD-1 antibody). After 4 days, the result was centrifuged at 2,000 rpm at 4°C for 3 minutes, and the supernatant culture solution was collected at 100 μL in a 96-well U-bottom plate and sealed and stored at -20°C.

14.2 Determination of change in IFN-γ secretion

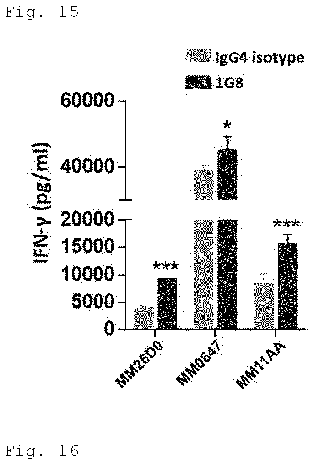

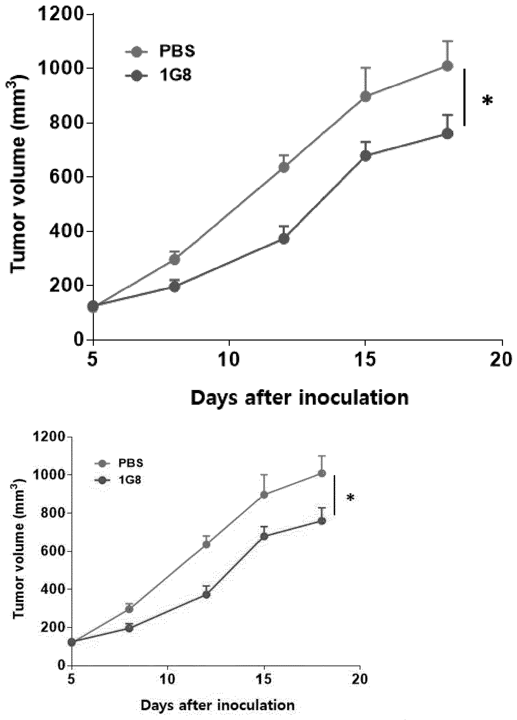

The culture medium stored at -20°C was allowed to thaw on the plate at room temperature. The amount of IFN-γ secretion was measured in accordance with the protocol of the BDTM cytometric bead array (CBA) human Th1/Th2/Th17 cytokine kit (BD, 560484). The lyophilized cytokine standard stock provided in the kit was transferred to a 15 mL tube, thawed by sufficiently pipetting with 2 mL of a diluent buffer, and then was allowed to stand at room temperature for at least 15 minutes. Then, the result was serially diluted at 1:1 with 300 μL of the same volume of buffer to prepare ten standards. A negative reference standard was prepared as a diluent buffer. The culture medium was diluted 1:20 using the diluent buffer of the kit. As many capture beads of each cytokine as the number of samples and standards were prepared, and 50 μL of the sample, 50 μL of the mixed capture bead sample, and 50 μL of the PE detection antibody were added to 96-well 1.1 mL Cluster Tubes Bulk (Axygen, MTS-11-C), followed by thorough mixing. The result was incubated in the dark for 3 hours, 300 μL of wash buffer was added thereto, centrifugation was performed at 4°C at 2,000 rpm for 3 minutes, and the supernatant was removed. The IFN-γ secretion of each sample after the process was assayed using LSR Fortessa and the result is shown in FIG. 15.

As can be seen from FIG. 15, the amount of IFN-γ that was secreted when anti-CD3/28 stimulation was applied to the PBMCs of multiple myeloma patients was significantly increased upon treatment with the TIGIT candidate antibody (1G8).

**Example 15. Determination of tumor growth inhibitory activity in human colorectal cancer cell xenograft model using humanized mice by TIGIT candidate antibody (1G8)**

In order to detect the tumor growth inhibitory effect of the TIGIT candidate antibody (1G8) *in vivo,* a xenograft animal model in which peripheral blood mononuclear cells from normal subjects and the human cell line were transplanted into immune-cell-knocked-out NOG (NOD/Shi-scid/IL-2Rγnull, Jackson Laboratory, 5-6 weeks old) mice was used. The human cancer cell line used herein was a human colon cancer cell line HT29 (ATCC, HTB-38), PVR expression was detected using a PE anti-PVR antibody (Thermo Fisher Scientific, 12-1550-41), and the results are shown in FIG. 16. PVR expressed in HT29 cells bound to TIGIT expressed in immune cells to induce activity of inhibition of immune cell activity. First, $3.5 \times 10^6$ cells/0.2 mL of HT29 cells were injected subcutaneously under the right foreleg of female NOG mice to induce tumor formation (Day 0). Six hours after tumor cell injection, Peripheral blood mononuclear cells were injected intraperitoneally at $7 \times 10^6$ cells/0.2 mL to create a mouse model having a human immune system. When the tumor volume grew to 50-80 mm$^3$, the tumor was classified into a group having a similar tumor size, and the TIGIT candidate antibody (1G8) (10 mg/kg) was administered intraperitoneally to mice twice a week, a total of 6 times. The average tumor volume of such an experimental group administered with the TIGIT candidate antibody (1G8) at a dose of 10 mg/kg exhibited tumor growth inhibitory activity of about 28.1% compared to the average tumor volume of the negative control group (*: P<0.05).

**Industrial Applicability**

It was found that the anti-TIGIT antibody or antigen-binding fragment thereof according to the present invention is specific to TIGIT, strongly binds thereto, and exhibits superior therapeutic efficacy compared to conventional anti-TIGIT antibodies. Therefore, the anti-TIGIT antibody or antigen-binding fragment thereof according to the present invention can be used as an immuno-oncology agent through immune cell activation.

In addition, the anti-TIGIT antibody or antigen-binding fragment thereof of the present invention can be used

**EP 3 985 025 A1**

in combination therapy with chemical drugs and other anticancer drugs.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

**Sequence Listing Free Text**

An electronic file is attached.

**Claims**

1. An antibody specifically binding to TIGIT (T-cell immunoreceptor with Ig and tyrosine-based inhibitory motif domains) or an antigen-binding fragment thereof, comprising:

   a heavy-chain CDR1 represented by SEQ ID NO: 3, 6, 9 or 12;
   a heavy-chain CDR2 represented by SEQ ID NO: 4, 7 or 10;
   a heavy-chain CDR3 represented by SEQ ID NO: 5, 8, 11 or 13;
   a light-chain CDR1 represented by SEQ ID NO: 14, 17, 20, 23 or 25;
   a light-chain CDR2 represented by SEQ ID NO: 15, 18, 21 or 26; and
   a light-chain CDR3 represented by SEQ ID NO: 16, 19, 22, 24 or 27.

2. The antibody or antigen-binding fragment thereof according to claim 1, wherein the antibody or antigen-binding fragment thereof comprises:

   a heavy-chain CDR1 of SEQ ID NO: 3, a heavy-chain CDR2 of SEQ ID NO: 4, a heavy-chain CDR3 of SEQ ID NO: 5, a light-chain CDR1 of SEQ ID NO: 14, a light-chain CDR2 of SEQ ID NO: 15, and a light-chain CDR3 of SEQ ID NO: 16;
   a heavy-chain CDR1 of SEQ ID NO: 6, a heavy-chain CDR2 of SEQ ID NO: 7, a heavy-chain CDR3 of SEQ ID NO: 8, a light-chain CDR1 of SEQ ID NO: 17, a light-chain CDR2 of SEQ ID NO: 18, and a light-chain CDR3 of SEQ ID NO: 19;
   a heavy-chain CDR1 of SEQ ID NO: 9, a heavy-chain CDR2 of SEQ ID NO: 10, a heavy-chain CDR3 of SEQ ID NO: 11, a light-chain CDR1 of SEQ ID NO: 20, a light-chain CDR2 of SEQ ID NO: 21, and a light-chain CDR3 of SEQ ID NO: 22;
   a heavy-chain CDR1 of SEQ ID NO: 9, a heavy-chain CDR2 of SEQ ID NO: 10, a heavy-chain CDR3 of SEQ ID NO: 11, a light-chain CDR1 of SEQ ID NO: 23, a light-chain CDR2 of SEQ ID NO: 21, and a light-chain CDR3 of SEQ ID NO: 24; or
   a heavy-chain CDR1 of SEQ ID NO: 12, a heavy-chain CDR2 of SEQ ID NO: 10, a heavy-chain CDR3 of SEQ ID NO: 13, a light-chain CDR1 of SEQ ID NO: 25, a light-chain CDR2 of SEQ ID NO: 26, and a light-chain CDR3 of SEQ ID NO: 27.

3. The antibody or antigen-binding fragment thereof according to claim 1, wherein the antibody or antigen-binding fragment thereof comprises at least one heavy-chain variable region selected from the group consisting of SEQ ID NOS: 28 to 32.

4. The antibody or antigen-binding fragment thereof according to claim 1, wherein the antibody or antigen-binding fragment thereof comprises at least one light-chain variable region selected from the group consisting of SEQ ID NOS: 34 to 38.

5. A nucleic acid encoding the antibody or antigen-binding fragment thereof according to any one of claims 1 to 4.

**6.** A vector comprising the nucleic acid according to claim 5.

**7.** A cell transformed with the vector according to claim 6.

**8.** A method of producing an antigen binding to TIGIT or an antigen-binding fragment thereof comprising:

    (a) culturing the cell according to claim 7; and
    (b) recovering an antibody or antigen-binding fragment thereof from the cultured cell.

**9.** A composition for preventing or treating cancer comprising the antibody or antigen-binding fragment thereof according to any one of claims 1 to 4 as an active ingredient.

**10.** A composition for preventing or treating cancer using the antibody or antigen-binding fragment thereof according to any one of claims 1 to 4 in combination with an anticancer therapy.

**11.** A composition for preventing or treating cancer using the antibody or antigen-binding fragment thereof according to any one of claims 1 to 4 in combination with an immune checkpoint inhibitor.

**12.** The composition according to claim 11, wherein the immune checkpoint inhibitor targets PD-1.

Fig. 1

**StandardCurve**

| 4-P Fit: y = (A - D)/( 1 + (x/C)^B ) + D: | A | B | C | D | E |
|---|---|---|---|---|---|
| 1B7 (Group01: Concentration vs Values) | 0.0499 | 0.778 | 13.2 | 2.41 | |
| 1B11 (Group02: Concentration vs Values) | 0.0283 | 0.733 | 3.31 | 1.66 | 0. |
| 1C3 (Group03: Concentration vs Values) | 0.00775 | 0.878 | 3.3 | 2.51 | 0. |
| 1E3 (Group04: Concentration vs Values) | 0.0461 | 0.848 | 7.66 | 0.525 | 0. |
| 1E4 (Group05: Concentration vs Values) | 0.000592 | 0.974 | 0.294 | 2.46 | 0. |
| 1E12 (Group06: Concentration vs Values) | -0.145 | 0.54 | 3.27 | 2.95 | 0. |
| 1F2 (Group07: Concentration vs Values) | -0.116 | 0.733 | 0.676 | 2.44 | 0. |
| 1F10 (Group08: Concentration vs Values) | 0.102 | 1.61 | 0.526 | 2.2 | 0. |
| 1G8 (Group09: Concentration vs Values) | 0.0034 | 1.03 | 0.203 | 2.37 | 0. |
| WIN-1B3 (Group10: Concentration vs Values) | -0.00289 | 0.337 | 582 | 1.84 | 0. |
| WIN-1B6 (Group11: Concentration vs Values) | 0.0702 | 1.22 | 4.75 | 1.47 | 0. |
| WIN-1B8 (Group12: Concentration vs Values) | 0.0898 | 1.31 | 9.07 | 0.486 | 0. |

Weighting: Fixed

**StandardCurve**

| 4-P Fit: y = (A - D)/( 1 + (x/C)^B ) + D: | A | B | C | D | R^2 |
|---|---|---|---|---|---|
| WIN-1B11 (Group01: Concentration vs Values) | 0.0611 | 1.15 | 2.86 | 2.19 | 0.992 |
| WIN-1C5 (Group02: Concentration vs Values) | 0.0511 | 0.77 | 47.9 | 2.04 | 0.999 |
| WIN-1C9 (Group03: Concentration vs Values) | 0.0215 | 0.636 | 12.7 | 1.79 | 0.998 |
| WIN-1D2 (Group04: Concentration vs Values) | 0.0411 | 0.886 | 4.39 | 2.24 | 0.999 |

Weighting: Fixed

**StandardCurve**

| 4-P Fit: y = (A - D)/( 1 + (x/C)^B ) + D: | A | B | C | D | E |
|---|---|---|---|---|---|
| 1B7 (Group01: Concentration vs Values) | 0.0499 | 0.778 | 13.2 | 2.41 | |
| 1B11 (Group02: Concentration vs Values) | 0.0283 | 0.733 | 3.31 | 1.66 | 0. |
| 1C3 (Group03: Concentration vs Values) | 0.00775 | 0.878 | 3.3 | 2.51 | 0. |
| 1E3 (Group04: Concentration vs Values) | 0.0461 | 0.848 | 7.66 | 0.525 | 0. |
| 1E4 (Group05: Concentration vs Values) | 0.000592 | 0.974 | 0.294 | 2.46 | 0. |
| 1E12 (Group06: Concentration vs Values) | -0.145 | 0.54 | 3.27 | 2.95 | 0. |
| 1F2 (Group07: Concentration vs Values) | -0.116 | 0.733 | 0.676 | 2.44 | 0. |
| 1F10 (Group08: Concentration vs Values) | 0.102 | 1.61 | 0.526 | 2.2 | 0. |
| 1G8 (Group09: Concentration vs Values) | 0.0034 | 1.03 | 0.203 | 2.37 | 0. |
| WIN-1B3 (Group10: Concentration vs Values) | -0.00289 | 0.337 | 582 | 1.84 | 0. |
| WIN-1B6 (Group11: Concentration vs Values) | 0.0702 | 1.22 | 4.75 | 1.47 | 0. |
| WIN-1B8 (Group12: Concentration vs Values) | 0.0898 | 1.31 | 9.07 | 0.486 | 0. |

Weighting: Fixed

**StandardCurve**

| 4-P Fit: y = (A - D)/( 1 + (x/C)^B ) + D: | A | B | C | D | R^2 |
|---|---|---|---|---|---|
| WIN-1B11 (Group01: Concentration vs Values) | 0.0611 | 1.15 | 2.86 | 2.19 | 0.992 |
| WIN-1C5 (Group02: Concentration vs Values) | 0.0511 | 0.77 | 47.9 | 2.04 | 0.999 |
| WIN-1C9 (Group03: Concentration vs Values) | 0.0215 | 0.636 | 12.7 | 1.79 | 0.998 |
| WIN-1D2 (Group04: Concentration vs Values) | 0.0411 | 0.886 | 4.39 | 2.24 | 0.999 |

Weighting: Fixed

Fig. 2

**StandardCurve**

| 4-P Fit: y = (A - D)/( 1 + (x/C)^B ) + D: | A | B | C | D | R^2 |
|---|---|---|---|---|---|
| 1C3 (Group02: Concentration vs Values) | 0.0738 | 1.49 | 3.56 | 1.03 | 0.999 |
| 1E4 (Group03: Concentration vs Values) | 0.0594 | 1.17 | 1.49 | 1.42 | 0.999 |
| 1E12 (Group04: Concentration vs Values) | 0.0551 | 0.908 | 2.94 | 0.884 | 0.999 |
| 1F2 (Group05: Concentration vs Values) | 0.0188 | 0.816 | 1.78 | 1.27 | 0.998 |
| 1F10 (Group06: Concentration vs Values) | 0.0565 | 1.05 | 5.35 | 2.2 | 1 |
| 1G8 (Group07: Concentration vs Values) | 0.0773 | 1.37 | 1.63 | 1.58 | 1 |
| WIN-1B6 (Group08: Concentration vs Values) | 0.121 | 1.71 | 0.861 | 0.929 | 0.971 |
| WIN-1B11 (Group09: Concentration vs Values) | 0.0842 | 1.33 | 1.63 | 1.36 | 0.999 |
| WIN-1D2 (Group10: Concentration vs Values) | 0.0926 | 1.22 | 1.75 | 1.31 | 0.998 |
| 1E8 (Group11: Concentration vs Values) | 0.122 | 1.32 | 1.73 | 0.814 | 0.986 |
| 1B11 (Group01: Concentration vs Values) | 0.073 | 1.34 | 4.21 | 0.636 | 0.999 |

Weighting: Fixed

Fig. 3

Fig. 4

Fig. 5

**1G8 IgG4**

**WIN-1B6 IgG4**

**Ref. Ab**

| Sample | ka (1/Ms) | kd (1/s) | KD (M) | Rmax (RU) | tc | Chi² (RU²) |
|---|---|---|---|---|---|---|
| Ref. Ab | 6.22E+05 | 2.33E-04 | 3.75E-10 | 52.54 | 2.85E+08 | 0.049 |
| 1G8 | 1.78E+06 | 1.0E-03 | 5.90E-10 | 62.88 | 8.01E+07 | 0.0574 |
| WIN-1B6 | 1.15E+06 | 2.458-02 | 2.14E-08 | 58.82 | 4.70E+07 | 0.0407 |

Fig. 6

| Name | EC50 (ug/ml) | Name | EC50 (ug/ml) |
|---|---|---|---|
| 1B11 | 0.041 | 1G8 | 0.017 |
| 1C3 | 0.053 | WIN_1B6 | 0.016 |
| 1E4 | 0.015 | WIN_1B11 | 0.021 |
| 1E12 | 0.068 | WIN_1D2 | 0.017 |
| 1F2 | 0.017 | 1E8 | 0.021 |
| 1F10 | 0.080 | | |

| Name | EC50 (ug/ml) | Name | EC50 (ug/ml) |
|---|---|---|---|
| 1B11 | 0.041 | 1G8 | 0.017 |
| 1C3 | 0.053 | WIN_1B6 | 0.016 |
| 1E4 | 0.015 | WIN_1B11 | 0.021 |
| 1E12 | 0.068 | WIN_1D2 | 0.017 |
| 1F2 | 0.017 | 1E8 | 0.021 |
| 1F10 | 0.080 | | |

Fig. 7

| Name | EC50 (ug/ml) | Name | EC50 (ug/ml) |
|---|---|---|---|
| 1B11 | 0.285 | 1G8 | 0.093 |
| 1C3 | 0.398 | WIN_1B6 | 0.068 |
| 1E4 | 0.082 | WIN_1B11 | 0.076 |
| 1E12 | 0.236 | WIN_1D2 | 0.071 |
| 1F2 | 0.143 | 1E8 | 0.102 |
| 1F10 | 0.380 | | |

Fig. 8

Fig. 9

**HEK293 Mouse TIGIT**

**HEK293 Monkey TIGIT**

**HEK293 Human TIGIT**

WIN_1B6 ⟶   WIN_1D2 ⟶   1E8 ⟶   1F10 ⟶   1G8 ⟶

**HEK293 Mouse TIGIT**

**HEK293 Monkey TIGIT**

**HEK293 Human TIGIT**

WIN_1B6 ⟶   WIN_1D2 ⟶   1E8 ⟶   1F10 ⟶   1G8 ⟶

Fig. 10

Fig. 11

A.

B.

A.

B.

Fig. 12a

Fig. 12b

Fig. 13

Fig. 14

Fig. 15

Fig. 16

<div style="text-align:center">**INTERNATIONAL SEARCH REPORT**</div>

| | International application No. |
|---|---|
| | **PCT/KR2020/006705** |

**A. CLASSIFICATION OF SUBJECT MATTER**

*C07K 16/28(2006.01)i, A61P 35/00(2006.01)i, A61K 39/00(2006.01)i*

According to International Patent Classification (IPC) or to both national classification and IPC

**B. FIELDS SEARCHED**

Minimum documentation searched (classification system followed by classification symbols)
C07K 16/28; A61K 39/00; A61K 39/395; A61P 35/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: TIGIT (T cell immunoreceptor with Ig and Tyrosine-based inhibitory motif domains), CDR, antibody, Phage display

**C. DOCUMENTS CONSIDERED TO BE RELEVANT**

| Category* | Citation of document, with indication, where appropriate, of the relevant passages | Relevant to claim No. |
|---|---|---|
| A | KR 10-2017-0041272 A (MERCK SHARP & DOHME CORP.) 14 April 2017<br>See claims 1-33; and paragraphs [0009]-[0058]. | 1-12 |
| A | KR 10-2018-0119653 A (JN BIOSCIENCES, LLC. et al.) 02 November 2018<br>See claims 12-17; and paragraphs [0061]-[0536]. | 1-12 |
| A | KR 10-2018-0053742 A (GENENTECH, INC.) 23 May 2018<br>See claims 25-40. | 1-12 |
| A | WO 2016-106302 A1 (BRISTOL-MYERS SQUIBB COMPANY) 30 June 2016<br>See claims 14-23; and paragraphs [0167]-[0201]. | 1-12 |
| A | WO 2018-102536 A1 (ONCOMED PHARMACEUTICALS, INC.) 07 June 2018<br>See claims 1-44; and paragraphs [0011]-[0177]. | 1-12 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 31 AUGUST 2020 (31.08.2020) | **01 SEPTEMBER 2020 (01.09.2020)** |

| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |
|---|---|

Form PCT/ISA/210 (second sheet) (January 2015)

# EP 3 985 025 A1

INTERNATIONAL SEARCH REPORT
Information on patent family members

International application No.

**PCT/KR2020/006705**

| Patent document cited in search report | Publication date | Patent family member | Publication date |
|---|---|---|---|
| KR 10-2017-0041272 A | 14/04/2017 | AP 201709765 A0 | 28/02/2017 |
| | | AP 201709765 D0 | 28/02/2017 |
| | | AU 2015-305754 A1 | 23/02/2017 |
| | | AU 2015-305754 A1 | 25/02/2016 |
| | | AU 2015-305754 B2 | 25/10/2018 |
| | | AU 2019-200426 A1 | 07/02/2019 |
| | | BR 112017003108 A2 | 05/12/2017 |
| | | CA 2957722 A1 | 25/02/2016 |
| | | CL 2017000310 A1 | 18/08/2017 |
| | | CL 2019001926 A1 | 29/11/2019 |
| | | CN 107148430 A | 08/09/2017 |
| | | CR 20170060 A | 18/04/2017 |
| | | DO P2017000046 A | 31/08/2017 |
| | | EA 201790413 A1 | 31/08/2017 |
| | | EP 3183267 A1 | 28/06/2017 |
| | | GT 201700033 A | 30/09/2019 |
| | | IL 250583 A | 30/04/2017 |
| | | IL 250583 D0 | 30/04/2017 |
| | | JP 2017-532007 A | 02/11/2017 |
| | | KR 10-2019-0133078 A | 29/11/2019 |
| | | KR 10-2050082 B1 | 29/11/2019 |
| | | MD 20170032 A2 | 31/08/2017 |
| | | MX 2017002229 A | 09/05/2017 |
| | | NI 201700019 A | 08/03/2017 |
| | | PE 02892017 A1 | 05/04/2017 |
| | | PE 20170289 A1 | 05/04/2017 |
| | | PH 12017500296 A1 | 28/06/2017 |
| | | SG 11201701161 A | 30/03/2017 |
| | | TN 2017000024 A1 | 04/07/2018 |
| | | TW 201609813 A | 16/03/2016 |
| | | US 10618958 B2 | 14/04/2020 |
| | | US 2016-0355589 A1 | 08/12/2016 |
| | | US 2017-198042 A1 | 13/07/2017 |
| | | US 2018-066055 A1 | 08/03/2018 |
| | | WO 2016-028656 A1 | 25/02/2016 |
| KR 10-2018-0119653 A | 02/11/2018 | AR 107804 A1 | 06/06/2018 |
| | | AU 2017-228474 A1 | 30/08/2018 |
| | | AU 2017-228474 A1 | 08/09/2017 |
| | | BR 112018067458 A2 | 02/01/2019 |
| | | CA 3014934 A1 | 08/09/2017 |
| | | CN 108883164 A | 23/11/2018 |
| | | EA 201891989 A1 | 30/04/2019 |
| | | EP 3423089 A1 | 09/01/2019 |
| | | IL 261188 A | 31/10/2018 |
| | | IL 261188 D0 | 31/10/2018 |
| | | JP 2019-513817 A | 30/05/2019 |
| | | MX 2018010485 A | 10/01/2019 |
| | | PH 12018501778 A1 | 15/05/2019 |

Form PCT/ISA/210 (patent family annex) (January 2015)

International application No.

**PCT/KR2020/006705**

| Patent document cited in search report | Publication date | Patent family member | Publication date |
|---|---|---|---|
| | | SG 11201806992 A | 27/09/2018 |
| | | TW 201734043 A | 01/10/2017 |
| | | US 10537633 B2 | 21/01/2020 |
| | | US 2017-281764 A1 | 05/10/2017 |
| | | UY 37141 A | 29/09/2017 |
| | | WO 2017-152088 A1 | 08/09/2017 |
| KR 10-2018-0053742 A | 23/05/2018 | AR 106146 A1 | 13/12/2017 |
| | | AU 2016-325610 A1 | 01/03/2018 |
| | | AU 2016-325610 A1 | 30/03/2017 |
| | | AU 2016-325610 B2 | 10/10/2019 |
| | | AU 2019-246814 A1 | 31/10/2019 |
| | | BR 112018005862 A2 | 16/10/2018 |
| | | CA 2994858 A1 | 30/03/2017 |
| | | CL 2018000744 A1 | 06/07/2018 |
| | | CN 108290946 A | 17/07/2018 |
| | | CO 2018004090 A2 | 30/11/2018 |
| | | CR 20180225 A | 09/07/2018 |
| | | EP 3353210 A2 | 01/08/2018 |
| | | HK 1258058 A1 | 01/11/2019 |
| | | IL 257636 A | 30/04/2018 |
| | | IL 257636 D0 | 30/04/2018 |
| | | JP 2018-532397 A | 08/11/2018 |
| | | JP 2020-074778 A | 21/05/2020 |
| | | KR 10-2020-0087283 A | 20/07/2020 |
| | | MX 2018003633 A | 01/08/2018 |
| | | NZ 739750 A | 29/11/2019 |
| | | PE 10462018 A1 | 03/07/2018 |
| | | PE 20181046 A1 | 03/07/2018 |
| | | PH 12018500608 A1 | 01/10/2018 |
| | | RU 2018114523 A | 25/10/2019 |
| | | RU 2018114523 A3 | 25/10/2019 |
| | | TW 201718644 A | 01/06/2017 |
| | | US 0017572 B2 | 10/07/2018 |
| | | US 0047158 B2 | 14/08/2018 |
| | | US 2017-088613 A1 | 30/03/2017 |
| | | US 2018-186875 A1 | 05/07/2018 |
| | | US 2019-119376 A1 | 25/04/2019 |
| | | WO 2017-053748 A2 | 30/03/2017 |
| | | WO 2017-053748 A3 | 11/05/2017 |
| | | ZA 201800941 B | 29/05/2019 |
| WO 2016-106302 A1 | 30/06/2016 | AR 103268 A1 | 26/04/2017 |
| | | AU 2015-369683 A1 | 10/08/2017 |
| | | AU 2015-369683 A1 | 30/06/2016 |
| | | BR 112017013385 A2 | 06/02/2018 |
| | | CA 2971732 A1 | 30/06/2016 |
| | | CL 2017001660 A1 | 23/03/2018 |
| | | CN 107207594 A | 26/09/2017 |
| | | CN 107207594 B | 07/05/2019 |

International application No.

**PCT/KR2020/006705**

| Patent document cited in search report | Publication date | Patent family member | Publication date |
|---|---|---|---|
| | | CN 110256558 A | 20/09/2019 |
| | | CO 2017006989 A2 | 05/01/2018 |
| | | EA 201791171 A1 | 30/11/2017 |
| | | EP 3237448 A1 | 01/11/2017 |
| | | IL 253013 A | 31/08/2017 |
| | | IL 253013 D0 | 31/08/2017 |
| | | JP 2017-520512 A | 27/07/2017 |
| | | JP 2018-035138 A | 08/03/2018 |
| | | JP 2020-062026 A | 23/04/2020 |
| | | JP 6180663 B2 | 16/08/2017 |
| | | JP 6633032 B2 | 22/01/2020 |
| | | KR 10-2017-0099966 A | 01/09/2017 |
| | | MX 2017007744 A | 05/09/2017 |
| | | PE 12442017 A1 | 24/08/2017 |
| | | PE 20171244 A1 | 24/08/2017 |
| | | PH 12017501166 A1 | 11/12/2017 |
| | | SG 11201705063 A | 28/07/2017 |
| | | TN 2017000267 A1 | 19/10/2018 |
| | | TW 201629102 A | 16/08/2016 |
| | | US 10189902 B2 | 29/01/2019 |
| | | US 2016-0176963 A1 | 23/06/2016 |
| | | US 2019-112375 A1 | 18/04/2019 |
| | | UY 36471 A | 30/06/2016 |
| | | WO 2016-106302 A9 | 30/06/2016 |
| | | ZA 201704981 B | 28/08/2019 |
| WO 2018-102536 A1 | 07/06/2018 | AU 2017-368155 A1 | 07/06/2018 |
| | | AU 2017-368155 A1 | 30/05/2019 |
| | | BR 112019010943 A2 | 01/10/2019 |
| | | CA 3044664 A1 | 07/06/2018 |
| | | CN 110662552 A | 07/01/2020 |
| | | EP 3548071 A1 | 09/10/2019 |
| | | IL 266847 A | 31/07/2019 |
| | | IL 266847 D0 | 31/07/2019 |
| | | JP 2020-503273 A | 30/01/2020 |
| | | KR 10-2019-0088057 A | 25/07/2019 |
| | | MX 2019006072 A | 14/08/2019 |
| | | US 2019-284269 A1 | 19/09/2019 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170088613

<110>    GREEN CROSS CORPORATION
         MOGAM BIOTECHNOLOGY RESEARCH INSTITUTE

<120>    Antibodies Against T cell Immunoreceptor with IgAntibodies

<130>    2410

<160>    39

<170>    PatentIn version 3.5

<210>    1
<211>    244
<212>    PRT
<213>    Artificial Sequence

<220>
<223>    Synthetic Sequence


<400>    1
Met Gly Trp Cys Leu Leu Leu Ile Trp Ala Gln Gly Leu Arg Gln Ala
 1               5                  10                  15

Pro Leu Ala Ser Gly Met Met Thr Gly Thr Ile Glu Thr Thr Gly Asn
                20                  25                  30

Ile Ser Ala Glu Lys Gly Gly Ser Ile Ile Leu Gln Cys His Leu Ser
                35                  40                  45

Ser Thr Thr Ala Gln Val Thr Gln Val Asn Trp Glu Gln Gln Asp Gln
        50                  55                  60

Leu Leu Ala Ile Cys Asn Ala Asp Leu Gly Trp His Ile Ser Pro Ser
65                  70                  75                  80

Phe Lys Asp Arg Val Ala Pro Gly Pro Gly Leu Gly Leu Thr Leu Gln
                85                  90                  95

Ser Leu Thr Val Asn Asp Ala Gly Glu Tyr Phe Cys Ile Tyr His Thr
                100                 105                 110

Tyr Pro Asp Gly Thr Tyr Thr Gly Arg Ile Phe Leu Glu Val Leu Glu
        115                 120                 125

Ser Ser Val Ala Glu His Gly Ala Arg Phe Gln Ile Pro Leu Leu Gly
        130                 135                 140

Ala Met Ala Ala Thr Leu Val Val Ile Cys Thr Ala Val Ile Val Val
145                 150                 155                 160

Val Ala Leu Thr Arg Lys Lys Lys Ala Leu Arg Ile His Ser Val Glu
                165                 170                 175

Gly Asp Leu Arg Arg Lys Ser Ala Gly Gln Glu Glu Trp Ser Pro Ser
        180                 185                 190

Ala Pro Ser Pro Pro Gly Ser Cys Val Gln Ala Glu Ala Ala Pro Ala
        195                 200                 205

Gly Leu Cys Gly Glu Gln Arg Gly Glu Asp Cys Ala Glu Leu His Asp
        210                 215                 220

24

```
Tyr Phe Asn Val Leu Ser Tyr Arg Ser Leu Gly Asn Cys Ser Phe Phe
225                 230             235                 240

Thr Glu Thr Gly
```

```
<210>    2
<211>    317
<212>    PRT
<213>    Artificial Sequence

<220>
<223>    Synthetic Sequence


<400>    2
Gly Asp Val Val Val Gln Ala Pro Thr Gln Val Pro Gly Phe Leu Gly
  1                 5                 10                15

Asp Ser Val Thr Leu Pro Cys Tyr Leu Gln Val Pro Asn Met Glu Val
             20                 25                 30

Thr His Val Ser Gln Leu Thr Trp Ala Arg His Gly Glu Ser Gly Ser
         35                 40                 45

Met Ala Val Phe His Gln Thr Gln Gly Pro Ser Tyr Ser Glu Ser Lys
        50                 55                 60

Arg Leu Glu Phe Val Ala Ala Arg Leu Gly Ala Glu Leu Arg Asn Ala
 65                 70                 75                 80

Ser Leu Arg Met Phe Gly Leu Arg Val Glu Asp Glu Gly Asn Tyr Thr
                85                 90                 95

Cys Leu Phe Val Thr Phe Pro Gln Gly Ser Arg Ser Val Asp Ile Trp
            100                105                110

Leu Arg Val Leu Ala Lys Pro Gln Asn Thr Ala Glu Val Gln Lys Val
            115                120                125

Gln Leu Thr Gly Glu Pro Val Pro Met Ala Arg Cys Val Ser Thr Gly
            130                135                140

Gly Arg Pro Pro Ala Gln Ile Thr Trp His Ser Asp Leu Gly Gly Met
145                 150                155                160

Pro Asn Thr Ser Gln Val Pro Gly Phe Leu Ser Gly Thr Val Thr Val
                165                170                175

Thr Ser Leu Trp Ile Leu Val Pro Ser Ser Gln Val Asp Gly Lys Asn
            180                185                190

Val Thr Cys Lys Val Glu His Glu Ser Phe Glu Lys Pro Gln Leu Leu
            195                200                205

Thr Val Asn Leu Thr Val Tyr Tyr Pro Pro Glu Val Ser Ile Ser Gly
            210                215                220

Tyr Asp Asn Asn Trp Tyr Leu Gly Gln Asn Glu Ala Thr Leu Thr Cys
225                 230                235                 240

Asp Ala Arg Ser Asn Pro Glu Pro Thr Gly Tyr Asn Trp Ser Thr Thr
```

25

```
                       245                    250                    255
        Met Gly Pro Leu Pro Pro Phe Ala Val Ala Gln Gly Ala Gln Leu Leu
                    260             265             270

        Ile Arg Pro Val Asp Lys Pro Ile Asn Thr Thr Leu Ile Cys Asn Val
                    275             280             285

        Thr Asn Ala Leu Gly Ala Arg Gln Ala Glu Leu Thr Val Gln Val Lys
                    290             295             300

        Glu Gly Pro Pro Ser Glu His Ser Gly Met Ser Arg Asn
        305             310             315


        <210>   3
        <211>   8
        <212>   PRT
        <213>   Artificial Sequence

        <220>
        <223>   Synthetic Sequence


        <400>   3
        Gly Phe Thr Phe Ser Asn Tyr Asn
          1               5


        <210>   4
        <211>   7
        <212>   PRT
        <213>   Artificial Sequence

        <220>
        <223>   Synthetic Sequence


        <400>   4
        Ser Ser Ser Ala Ser Tyr Ile
          1               5


        <210>   5
        <211>   7
        <212>   PRT
        <213>   Artificial Sequence

        <220>
        <223>   Synthetic Sequence


        <400>   5
        Asp Glu Gly Ser Arg Asp Ser
          1               5


        <210>   6
        <211>   8
        <212>   PRT
        <213>   Artificial Sequence

        <220>
```

26

```
<223>      Synthetic Sequence


<400>      6
Gly Tyr Thr Phe Thr Ser Tyr Tyr
  1                   5


<210>      7
<211>      8
<212>      PRT
<213>      Artificial Sequence

<220>
<223>      Synthetic Sequence


<400>      7
Ile Asn Pro Ser Gly Gly Ser Thr
  1                   5


<210>      8
<211>      7
<212>      PRT
<213>      Artificial Sequence

<220>
<223>      Synthetic Sequence


<400>      8
Ala Ser Arg Ser Gly Ser Gly
  1                   5


<210>      9
<211>      8
<212>      PRT
<213>      Artificial Sequence

<220>
<223>      Synthetic Sequence


<400>      9
Gly Tyr Thr Phe Thr Ser Tyr Gly
  1                   5


<210>      10
<211>      8
<212>      PRT
<213>      Artificial Sequence

<220>
<223>      Synthetic Sequence


<400>      10
Ile Ser Ala Tyr Asn Gly Asn Thr
  1                   5
```

```
<210>      11
<211>      4
<212>      PRT
<213>      Artificial Sequence

<220>
<223>      Synthetic Sequence


<400>      11
Ala Arg Ala Gly
  1


<210>      12
<211>      8
<212>      PRT
<213>      Artificial Sequence

<220>
<223>      Synthetic Sequence


<400>      12
Gly Tyr Thr Phe Ser Ser Tyr Ala
  1               5


<210>      13
<211>      5
<212>      PRT
<213>      Artificial Sequence

<220>
<223>      Synthetic Sequence


<400>      13
Ala Arg Val Asp Phe
  1               5


<210>      14
<211>      6
<212>      PRT
<213>      Artificial Sequence

<220>
<223>      Synthetic Sequence


<400>      14
Gln Ser Ile Ser Arg Tyr
  1               5


<210>      15
<211>      7
<212>      PRT
<213>      Artificial Sequence

<220>
```

```
<223>      Synthetic Sequence


<400>      15
Gly Ala Ser Ser Leu Gln Ser
 1               5


<210>      16
<211>      11
<212>      PRT
<213>      Artificial Sequence

<220>
<223>      Synthetic Sequence


<400>      16
Gln Gln Ser Tyr Thr Thr Pro Gly Ala Phe Thr
 1               5                   10


<210>      17
<211>      7
<212>      PRT
<213>      Artificial Sequence

<220>
<223>      Synthetic Sequence


<400>      17
Gln Ser Val Ser Ser Ser Tyr
 1               5


<210>      18
<211>      7
<212>      PRT
<213>      Artificial Sequence

<220>
<223>      Synthetic Sequence


<400>      18
Gly Ala Ser Ser Arg Ala Thr
 1               5


<210>      19
<211>      10
<212>      PRT
<213>      Artificial Sequence

<220>
<223>      Synthetic Sequence


<400>      19
Gln Gln Tyr Gly Ser Ser Pro Gly Gly Thr
 1               5                   10
```

```
<210>    20
<211>    6
<212>    PRT
<213>    Artificial Sequence

<220>
<223>    Synthetic Sequence


<400>    20
Gln Thr Ile Arg Ser Tyr
  1               5


<210>    21
<211>    7
<212>    PRT
<213>    Artificial Sequence

<220>
<223>    Synthetic Sequence


<400>    21
Ala Ala Ser Ser Leu Gln Ser
  1               5


<210>    22
<211>    10
<212>    PRT
<213>    Artificial Sequence

<220>
<223>    Synthetic Sequence


<400>    22
Gln Gln Ser Tyr Ser Thr Leu Pro Leu Thr
  1               5                   10


<210>    23
<211>    6
<212>    PRT
<213>    Artificial Sequence

<220>
<223>    Synthetic Sequence


<400>    23
Gln Ser Ile Ser Ser Tyr
  1               5


<210>    24
<211>    10
<212>    PRT
<213>    Artificial Sequence

<220>
```

```
<223>    Synthetic Sequence


<400>    24
Gln Gln Ser Tyr Ser Thr Leu Ala Ile Thr
 1               5                   10


<210>    25
<211>    6
<212>    PRT
<213>    Artificial Sequence

<220>
<223>    Synthetic Sequence


<400>    25
Gln Gly Ile Ser Asn Tyr
 1               5


<210>    26
<211>    7
<212>    PRT
<213>    Artificial Sequence

<220>
<223>    Synthetic Sequence


<400>    26
Ala Ala Ser Thr Leu Gln Ser
 1               5


<210>    27
<211>    9
<212>    PRT
<213>    Artificial Sequence

<220>
<223>    Synthetic Sequence


<400>    27
Gln Lys Ser Asn Ser Ala Pro Leu Thr
 1               5


<210>    28
<211>    129
<212>    PRT
<213>    Artificial Sequence

<220>
<223>    Synthetic Sequence


<400>    28
Glu Val Gln Leu Val Glu Ser Gly Gly Gly Leu Val Lys Pro Gly Gly
 1               5                   10                  15
```

```
Ser Leu Arg Ile Ser Cys Ala Ala Ser Gly Phe Thr Phe Ser Asn Tyr
            20                  25                  30

Asn Met Ile Trp Val Arg Gln Ala Pro Gly Lys Gly Leu Glu Trp Val
            35                  40                  45

Ser Ser Ile Ser Ser Ser Ala Ser Tyr Ile Tyr Tyr Ala Asp Ser Val
        50                  55                  60

Lys Gly Arg Phe Thr Ile Ser Arg Asp Asn Ala Lys Asn Ser Leu Tyr
    65                  70                  75                  80

Leu Gln Met Asn Ser Leu Arg Ala Glu Asp Thr Ala Val Tyr Tyr Cys
                85                  90                  95

Ala Arg Asp Glu Gly Ser Arg Asp Ser Trp Asn Asn Gly Pro Tyr Tyr
            100                 105                 110

Tyr Ser Gly Met Asp Val Trp Gly Gln Gly Thr Thr Val Thr Val Ser
        115                 120                 125

Ser
```

```
<210>    29
<211>    121
<212>    PRT
<213>    Artificial Sequence

<220>
<223>    Synthetic Sequence

<400>    29
Glu Val Gln Leu Val Gln Ser Gly Ala Glu Val Lys Lys Pro Gly Ala
    1                   5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
            35                  40                  45

Gly Ile Ile Asn Pro Ser Gly Gly Ser Thr Ser Tyr Ala Gln Lys Phe
        50                  55                  60

Gln Gly Arg Val Thr Met Thr Arg Asp Thr Ser Thr Ser Thr Val Tyr
    65                  70                  75                  80

Met Glu Leu Ser Ser Leu Arg Ser Glu Asp Thr Ala Val Tyr Tyr Cys
                85                  90                  95

Ala Ser Arg Ser Gly Ser Gly Trp Phe Gly Ala Leu Asp Tyr Trp Gly
            100                 105                 110

Gln Gly Thr Leu Val Thr Val Ser Ser
        115                 120
```

```
<210>    30
<211>    120
<212>    PRT
```

<210> 213 Artificial Sequence

<220>
<223> Synthetic Sequence

<400> 30
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
            35                  40                  45

Gly Trp Ile Ser Ala Tyr Asn Gly Asn Thr Asn Tyr Ala Gln Lys Leu
        50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Ala Gly Trp Glu Gln Gln Leu Gly Phe Asp Tyr Trp Gly Gln
            100                 105                 110

Gly Thr Leu Val Thr Val Ser Ser
        115                 120


<210> 31
<211> 120
<212> PRT
<213> Artificial Sequence

<220>
<223> Synthetic Sequence


<400> 31
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
            35                  40                  45

Gly Trp Ile Ser Ala Tyr Asn Gly Asn Thr Asn Tyr Ala Gln Lys Leu
        50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Ala Gly Trp Glu Gln Gln Leu Gly Phe Asp Tyr Trp Gly Gln
            100                 105                 110

```
Gly Thr Leu Val Thr Val Ser Ser
        115                 120


<210>    32
<211>    121
<212>    PRT
<213>    Artificial Sequence

<220>
<223>    Synthetic Sequence


<400>    32
Gln Val Gln Leu Val Gln Ser Gly Thr Glu Val Lys Lys Pro Gly Ala
  1                 5                  10                  15

Ser Val Lys Val Ser Cys Lys Ala Ser Gly Tyr Thr Phe Ser Ser Tyr
            20                  25                  30

Ala Ile Thr Trp Val Arg Gln Ala Pro Gly Gln Gly Leu Glu Trp Met
            35                  40                  45

Gly Trp Ile Ser Ala Tyr Asn Gly Asn Thr Asn Tyr Ala Gln Lys Leu
        50                  55                  60

Gln Gly Arg Val Thr Met Thr Thr Asp Thr Ser Thr Ser Thr Ala Tyr
 65                  70                  75                  80

Met Glu Leu Arg Ser Leu Arg Ser Asp Asp Thr Ala Val Tyr Tyr Cys
                85                  90                  95

Ala Arg Val Asp Phe Trp Ser Gly Tyr Asn Tyr Phe Asp Tyr Trp Gly
            100                 105                 110

Gln Gly Thr Leu Val Thr Val Ser Ser
        115                 120


<210>    33
<211>    126
<212>    PRT
<213>    Artificial Sequence

<220>
<223>    Synthetic Sequence


<400>    33
Glu Val Gln Leu Gln Gln Ser Gly Pro Gly Leu Val Lys Pro Ser Gln
  1                 5                  10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
            35                  40                  45

Trp Leu Gly Lys Thr Tyr Tyr Arg Phe Lys Trp Tyr Ser Asp Tyr Ala
        50                  55                  60

Val Ser Val Lys Gly Arg Ile Thr Ile Asn Pro Asp Thr Ser Lys Asn
 65                  70                  75                  80
```

34

```
Gln Phe Ser Leu Gln Leu Asn Ser Val Thr Pro Glu Asp Thr Ala Val
                85                  90                  95

Phe Tyr Cys Thr Arg Glu Ser Thr Thr Tyr Asp Leu Leu Ala Gly Pro
            100                 105                 110

Phe Asp Tyr Trp Gly Gln Gly Thr Leu Val Thr Val Ser Ser
        115                 120                 125
```

```
<210>    34
<211>    110
<212>    PRT
<213>    Artificial Sequence

<220>
<223>    Synthetic Sequence


<400>    34
Ala Ile Gln Met Thr Gln Ser Pro Ser Ser Leu Ser Ala Ser Val Gly
  1               5                  10                  15

Asp Arg Val Thr Ile Thr Cys Arg Ala Ser Gln Ser Ile Ser Arg Tyr
            20                  25                  30

Leu Asn Trp Tyr Gln His Lys Pro Gly Lys Ala Pro Lys Leu Leu Ile
        35                  40                  45

Tyr Gly Ala Ser Ser Leu Gln Ser Gly Val Pro Ser Arg Phe Arg Gly
    50                  55                  60

Ser Gly Ser Gly Thr Asp Phe Thr Leu Thr Ile Ser Ser Leu Gln Pro
 65                 70                  75                  80

Glu Asp Phe Ala Thr Tyr Tyr Cys Gln Gln Ser Tyr Thr Thr Pro Gly
            85                  90                  95

Ala Phe Thr Phe Gly Gly Gly Thr Lys Val Glu Ile Lys Arg
            100                 105                 110
```

```
<210>    35
<211>    110
<212>    PRT
<213>    Artificial Sequence

<220>
<223>    Synthetic Sequence


<400>    35
Glu Ile Val Leu Thr Gln Ser Pro Gly Thr Leu Ser Leu Ser Pro Gly
  1               5                  10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45
```

```
Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Gly Gly Thr Phe Gly Gln Gly Thr Lys Val Glu Ile Lys Arg
                100                 105                 110


<210>    36
<211>    109
<212>    PRT
<213>    Artificial Sequence

<220>
<223>    Synthetic Sequence


<400>    36
Ala Ile Gln Leu Thr Gln Ser Pro Ser Ser Leu Ser Ala Ser Val Gly
    1                5                  10                  15

Asp Arg Val Thr Ile Ser Cys Arg Ala Ser Gln Thr Ile Arg Ser Tyr
                20                  25                  30

Leu Asn Trp Tyr Gln Gln Lys Pro Gly Lys Ala Pro Lys Leu Leu Ile
            35                  40                  45

Tyr Ala Ala Ser Ser Leu Gln Ser Gly Val Pro Leu Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Leu Pro
                85                  90                  95

Leu Thr Phe Gly Gly Gly Thr Lys Val Glu Ile Lys Arg
            100                 105


<210>    37
<211>    109
<212>    PRT
<213>    Artificial Sequence

<220>
<223>    Synthetic Sequence


<400>    37
Ala Ile Gln Leu Thr Gln Ser Pro Ser Ser Leu Ser Ala Ser Val Gly
    1                5                  10                  15

Asp Arg Val Thr Ile Thr Cys Arg Ala Ser Gln Ser Ile Ser Ser Tyr
                20                  25                  30

Leu Asn Trp Tyr Gln Gln Lys Pro Gly Lys Ala Pro Lys Leu Leu Ile
            35                  40                  45
```

```
        Tyr Ala Ala Ser Ser Leu Gln Ser Gly Val Pro Ser Arg Phe Ser Gly
            50                  55                  60

        Ser Gly Ser Gly Thr Asp Phe Thr Leu Thr Ile Ser Ser Leu Gln Pro
            65                  70                  75                  80

        Glu Asp Phe Ala Thr Tyr Tyr Cys Gln Gln Ser Tyr Ser Thr Leu Ala
                        85                  90                  95

        Ile Thr Phe Gly Gln Gly Thr Arg Leu Glu Ile Lys Arg
                        100                 105


        <210>   38
        <211>   108
        <212>   PRT
        <213>   Artificial Sequence

        <220>
        <223>   Synthetic Sequence


        <400>   38
        Asp Ile Gln Met Thr Gln Ser Pro Ser Ser Leu Ser Ala Ser Val Gly
            1                   5                   10                  15

        Asp Arg Val Thr Ile Thr Cys Arg Ala Ser Gln Gly Ile Ser Asn Tyr
                        20                  25                  30

        Leu Ala Trp Tyr Gln Gln Lys Pro Gly Lys Val Pro Lys Val Leu Ile
                        35                  40                  45

        Tyr Ala Ala Ser Thr Leu Gln Ser Gly Val Pro Ser Arg Phe Ser Gly
            50                  55                  60

        Ser Gly Ser Gly Thr Asp Phe Thr Leu Thr Ile Ser Ser Leu Gln Pro
            65                  70                  75                  80

        Glu Asp Val Ala Thr Tyr Tyr Cys Gln Lys Ser Asn Ser Ala Pro Leu
                        85                  90                  95

        Thr Phe Gly Gly Gly Thr Lys Val Glu Ile Lys Arg
                        100                 105


        <210>   39
        <211>   114
        <212>   PRT
        <213>   Artificial Sequence

        <220>
        <223>   Synthetic Sequence


        <400>   39
        Asp Ile Val Met Thr Gln Ser Pro Asp Ser Leu Ala Val Ser Leu Gly
            1                   5                   10                  15

        Glu Arg Ala Thr Ile Asn Cys Lys Ser Ser Gln Thr Val Leu Tyr Ser
                        20                  25                  30

        Ser Asn Asn Lys Lys Tyr Leu Ala Trp Tyr Gln Gln Lys Pro Gly Gln
```

```
              35                    40                       45

      Pro Pro Asn Leu Leu Ile Tyr Trp Ala Ser Thr Arg Glu Ser Gly Val
          50                    55                  60

      Pro Asp Arg Phe Ser Gly Ser Gly Ser Gly Thr Asp Phe Thr Leu Thr
          65                70                  75                    80

      Ile Ser Ser Leu Gln Ala Glu Asp Val Ala Val Tyr Tyr Cys Gln Gln
                      85                  90                    95

      Tyr Tyr Ser Thr Pro Phe Thr Phe Gly Pro Gly Thr Lys Val Glu Ile
              100                   105                 110

      Lys Arg
```